(19) Europäisches Patentamt
European Patent Office
Office européen des brevets

(11) Publication number: **0 140 540 B1**

(12) **EUROPEAN PATENT SPECIFICATION**

(45) Date of publication of patent specification: **09.05.90**

(21) Application number: **84306030.2**

(22) Date of filing: **03.09.84**

(51) Int. Cl.[5]: **C 07 D 495/04,**
C 07 D 493/04,
C 07 D 409/06,
C 07 D 307/34,
C 07 D 307/79, G 03 C 1/72 //
C07D333/24, C07D307/54,
C07D333/56, C07D333/60,
C07D333/38, C07D333/22
,(C07D495/04, 333:00,
307:00),(C07D493/04, 307:00,
307:00),(C07D495/04, 333:00,
209:00)

(54) **Photochromic compounds and their uses in photoreactive lenses.**

(30) Priority: **07.09.83 US 530161**

(43) Date of publication of application:
**08.05.85 Bulletin 85/19**

(45) Publication of the grant of the patent:
**09.05.90 Bulletin 90/19**

(84) Designated Contracting States:
**AT BE CH DE FR IT LI LU NL SE**

(56) References cited:
**FR-A-2 363 568**
**GB-A-1 442 628**
**GB-A-1 464 603**
**GB-A-1 600 615**
**GB-A-1 602 755**
**GB-A-2 002 752**

(73) Proprietor: **THE PLESSEY COMPANY plc**
**Vicarage Lane**
**Ilford Essex, IG1 4AQ (GB)**

(72) Inventor: **Heller, Harry G.**
**135 Pencisely Road**
**Llandaff Cardiff, CF5 1DL (GB)**
Inventor: **Oliver, Stephen N.**
**47 Pen-y-cae Mornington Meadows**
**Caerphilly Mid-Glamorgan Wales (GB)**
Inventor: **Whittal, John**
**19 Pen-yr-allt Watford Park Caerphilly**
**Mid-Glamorgan Wales (GB)**
Inventor: **Johncock, William**
**48 Avenue Eugene Lancy**
**CH-1212 Grand-Lancy Geneva (CH)**
Inventor: **Darcy, Paul J.**
**Buarth Road**
**Aberystwyth Wales (GB)**
Inventor: **Trundle, Clive**
**27 Hillside Avenue Silverstone**
**Towcester Northants (GB)**

(74) Representative: **Woodcraft, David Charles**
**BROOKES & MARTIN High Holborn House 52/54**
**High Holborn**
**London, WC1V 6SE (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

Background of the Invention

This invention relates to photochromic compounds and is particularly concerned with compounds having the ability to undergo a change to a coloured or more strongly coloured form in strong sunlight and revert to their original form on removal or reduction of the U.V. component of sunlight, e.g. in diffuse daylight conditions.

Description of the Prior Art

In general, known photochromic compounds absorb U.V. light to generate coloured forms but these tend to revert to the colourless form when exposed to white light and also undergo thermal fade at ambient temperatures. U.K. Patent Specification No. 2,002,752 discloses a series of photochromic compounds having a high degree of photosensitivity so that they colour in U.V. light and reverse rapidly in white light. Such compounds are not suitable sunglasses or related applications because of this tendency to revert to the colourless form in white light. As a consequence, they show little or no colouring in unfiltered sunlight.

While it is possible to select compounds which have a low quantum efficiency for bleaching in white light this characteristic is usually associated with a coloured form which is thermally relatively stable so that reversion to the colourless form at normal ambient temperatures is very slow. As a result, such compounds would not be suitable in photo-reactive lenses.

A new class of photochromic compounds has been discovered which possess the combination of properties necessary for photoreactive lenses, namely (a) a high quantum efficiency for colouring in the near ultra-violet (b) a low quantum yield for bleaching with visible white light and (c) fast thermal fade at ambient temperatures, but not so rapid that the combination of white light bleaching and thermal fade prevent colouring by the ultraviolet component of strong sunlight. In addition, these properties are desirably retained in the conventional rigid plastics used for ophthalmic and plano lenses.

The term "heliochromic" is introduced to describe the behaviour of these novel compounds and will be used for this purpose in the remainder of this specification.

Summary of the Invention

According to one aspect of the present invention, there is provided a series of photochromic compounds of the general formula:

(I)

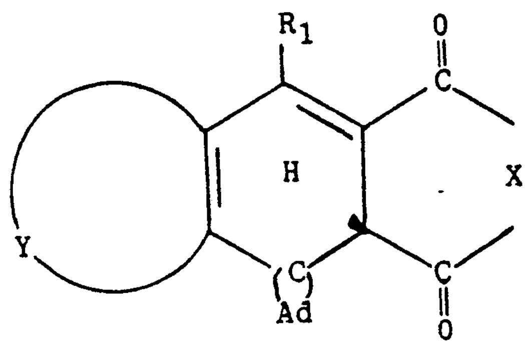

Ad⌣C< represents an adamantylidene or a substituted adamantylidene group;

$R_1$ represents hydrogen, alkyl, aryl, aralkyl or a heterocyclic group;

X represents oxygen or <$NR_2$, in which $R_2$ is hydrogen, alryl, alkyl, or aralkyl and

(II)

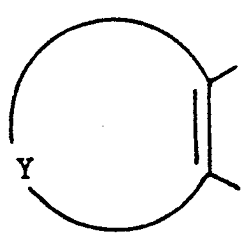

represents an aromatic group, an unsaturated heterocyclic group or a benzannelated heterocyclic group.

Examples of possible substituents in the adamantylidene group are halogen and hydroxy.

Preferred classes of heliochromic compounds falling within formula (I) are those in which the group represented by the formula (II):

(II)

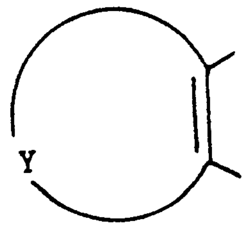

is a resonance stabilised heterocyclic group, containing an oxygen or sulphur heteroatom e.g. furyl or thienyl. Such heterocyclic groups may be benzannelated and substituted in positions other than where the group is linked to the six-membered group in formula (I), especially the α-position. Suitable substituents are deuterium, lower alkyl, e.g. methyl, ethyl, propyl, aryl (e.g. phenyl), nitro, and halogen, (e.g. chloro or bromo) groups.

Examples of aromatic or heterocyclic groups represented by the above formula (II) are groups derived from 2,5-, 2,4- or 3,5-dimethoxyphenyl; 2- or 3-furyl; or 2- or 3-thienyl groups, which may be substituted or benzannelated by cyclisation onto that group with a subsequent hydrogen shift.

The introduction of electron-withdrawing groups such as bromine in the furyl or thienyl group will cause a hypsochromic colour shift in the corresponding heliochromic compound. Also, the introduction of substituents (including benzannelation) in the furyl and thienyl ring tends to increase the resistance of the compounds to oxidation and thereby reduces the fatigue rate. Because fatigue reactions appear to proceed by an oxidative mechanism, it is also desirable to protect the compounds from oxygen in their ultimate application, e.g. by encapsulation or by use of oxygen scavengers, when employed in photoreactive lens manufacture.

The group represented by $R_1$ in the above formula (I) is preferably other than hydrogen e.g. substituted with a lower alkyl, i.e. methyl, ethyl, propyl or butyl (especially methyl) or phenyl. Preferred groups represented by $R_2$ are methyl and phenyl.

Detailed Description of the Invention

In the following description, reference will be made to the accompanying sheets of formulae drawings.

Heliochromic behaviour and compounds having structures falling within the general formula (I) above were discovered as a consequence of investigation of the effect of heating of fulgides. As noted in British Patent Specification No. 2,002,752, certain fulgides can be converted into their coloured forms by heating. The reaction was found to be reversible using white light and the coloured form was found to be stable in the dark at room temperature. No value was perceived in these fulgides for sunglass applications because their photochromic response to sunlight was generally poor.

Nevertheless, these remarkable photochromic properties prompted further investigation of the reactions because of the present inventors' interest in the fundamental chemistry of fulgides and their corresponding coloured forms. As a consequence of these investigations, it was found that certain fulgides containing an adamantylidene group underwent a photochemical or thermal ring closure and a thermal 1,5-hydrogen shift to give compounds which surprisingly exhibited heliochromic properties. For example, in the case of adamantylidene [α-3-thienyl)ethylidene] succinic anhydride (structure 7 in sheet 1 of the accompanying drawings) heating at about 140°C, caused ring closure to the coloured cyclic form (structure 8) which underwent a 1,5-hydrogen shift to the cyclic structure (9) (particularly at temperatures of about 180°C). While fulgide (7) is not heliochromic, the colourless cyclic structure (9) is heliochromic. On irradiation with ultraviolet light in solution in toluene (even in the presence of white light), it gives the purple/blue coloured form (structure 10) which reverses to the colourless form (structure 9) at ambient temperatures in the absence of u.v. stimulation. Because the coloured form (10) exhibits such a very slow white light reversal, coupled with a relatively fast thermal fade at ambient temperatures, it is potentially valuable for use in photoreactive lenses.

The adamantylidene group possesses a rigid strain free cage structure. Its structure is shown in the accompanying formula drawings. For convenience, the informal shorthand form, Ad⁀C is used in this specification to designate the adamantylidene group as indicated at the foot of sheets 3 to 10 of the formula drawings. It is believed that the adamantylidene group plays a vital part in the generation of the heliochromic properties of this invention, because this bulky, stable group weakens the single bond of the six-membered ring of which it forms part, facilitating electrocyclic ring opening on irradiation with ultraviolet light. Attempts have been made to substitute other bulky groups for the adamantylidene group but there appear to be few practical alternative groups. For example, camphor and fenchone would appear at first sight to be suitable alternatives but these ketones failed to undergo the Stobbe reaction, probably because of steric hindrance. Simpler acyclic, cyclic and bicyclic ketones do undergo the Stobbe reaction and the corresponding half esters can be hydrolysed to the diacids and hence to the corresponding

fulgides. Although the latter are often photochromic, heating does not produce a heliochromic derivative.

The adamantylidene group need not be attached to the group

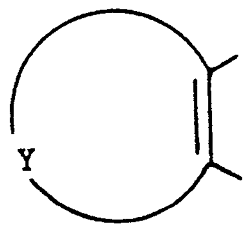

where this represents a heterocylic group, via the carbon atom adjacent to the heteroatom. For example, referring to structural formula 9 (sheet 1 of the drawings) the adamantylidene group need not be attached to the thienyl group via the 2-position but could equally be attached via the 3-position as shown in structural formula (9A).

The preparation of the fulgides from which the heliochromes are obtained is conveniently carried out using a dialkyl adamantylidenesuccinate (preferably the diethyl ester) as the starting material. The preparation of this ester is described in U.K. Patent Specification No. 2,051,813 and can be obtained in over 80% yield and more than 98% purity. The reaction scheme on sheet 2 in the accompanying drawings indicates the reaction and the structure of the diester (structure 11, where R = Et). Preferred reaction conditions involve condensation of adamantanone with diethyl succinate in the presence of a condensing agent, such as sodium hydride (as 50% dispersion in oil) or potassium t-butoxide (in t-butanol), followed by esterification of the half ester (structure 11), where R = H) using an ethanol/toluene mixture and toluene-p-sulfonic acid as catalyst. The adamantylidene diester is condensed with the appropriate aldehyde or ketone

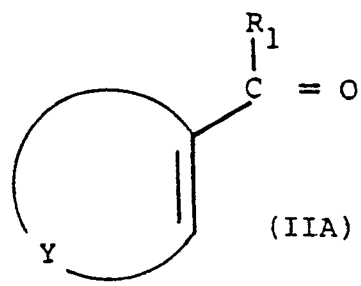

(IIA)

wherein $R_1$ has the same significance as in formula (I) above, in the presence of a condensing agent, as before, followed by hydrolysis of the resulting half ester (structure 12) to the diacids, e.g. with 10% ethanolic potassium hydroxide solution.

The E-diacid is obtained in admixture with the Z-isomer, by hydrolysis, e.g. with 10% ethanolic potassium hydroxide solution. The diacids are cyclised with an appropriate agent, e.g. acetic anhydride or acetyl chloride to form the corresponding fulgides. The heliochromic rearrangement product is obtained by heating the fulgides or fulgimides or by a combination of heating and irradiation with U.V. light or by Lewis acid-catalysed cyclisation, e.g using $SnCl_4$. A combination of U.V. irradiation and heating has the advantage that a lower temperature can be employed and under such milder conditions a better yield can be anticipated. Where the rearrangement is effected by heating alone, a temperature of about 180°C is required. On the other hand, irradiation with U.V. light is effective to promote rearrangement at temperatures as low as about 25°C. The mixture of diacids can be separated (e.g. via the sparingly soluble potassium salt of the E-diacid) but this is generally unnecessary since the Z-fulgide is converted into E-fulgide on heating. Accordingly the mixture can be heated to form the heliochromic compound.

In cases where the thiophen starting materials of general formula (IIA) above is not readily obtainable, this may be synthesised by a Gewald type reaction in which an acetaldehyde derivative is condensed with sulphur and ethyl cyanoacetate, preferably in the presence of morpholine and a solvent such as ethanol. The product of this reaction is an amino ester of a thiophen derivative. If the acetaldehyde derivative has an aryl substituent, this will be present as a substituent in the thiophene ring. The thiophene derivative is converted to the corresponding acid chloride by deamination and hydrolysis. The resultant acid chloride is reacted with a salt (e.g. the magnesium salt) of diethyl malonate to yield the corresponding ketone. This process which is a novel procedure is illustrated in Example 18 and Sheet 13 of the formula drawings. Referring to Sheet 13, this illustrates the preparation of 4-acetyl-2-phenylthiophene (6) starting from phenylacetaldehyde (1). Derivatives of compound (6) in which the phenyl group is substituted, e.g. with alkoxy or alkyl, are readily obtained by selection of an appropriate substituted phenyl acetaldehyde starting compound (1). 4 acetyl-2-phenylthiophene, and related compounds in which the phenyl group is substituted are novel compounds.

The following Examples are given to illustrate the preparation of heliochromic compounds in accordance with the invention. Reaction schemes showing the formation of the heliochromic compounds from their respective fulgide precursor are given in the accompanying sheets 1 to 13, inclusive, identified as reaction schemes 1 to 11. In all cases where it is stated that compounds were irradiated with "white" light, an intense white light source, containing a U.V. component, was used.

Example 1

Scheme 1 R=Ph (Sheet 3)

2-Phenyl-4-acetylthiophen (8 g) was condensed with either diethyl or dimethyl adamantylidenesuccinate (18 g) in toluene (100 $cm^3$), using sodium hydride (6 g) as condensing agent, and gave, after work up and hydrolysis with a slight excess of 10% ethanolic potassium hydroxide solution, the E-diacid as the potassium salt. The diacid, obtained by acidification with hydrochloric acid, was cyclised with acetyl chloride (50 $cm^3$) and gave the fulgide of structure (1) as yellow crystals (4.7 g).

On heating the fulgide (1) (4.7 g) at 180°C in o-dichlorobenzene it cyclised to the coloured 7,7a-dihydro-benzothiophen (7,7a-DHBT) (2) which rearranged to the heliochromic compound (3) (4.4 g) 94% yield, after crystallisation from o-dichlorobenzene. A blue coloured form (4) was obtained by irradiation with white light which reverted rapidly to the colourless form (3) on removal of the white light.

Example 2

Scheme 1 R=H (Sheet 3)

3-Acetylthiophen (50 g) was condensed with diethyl adamantylidenesuccinate (142 g) in toluene (400 $cm^3$) using sodium hydride (44 g) as condensing agent and gave, after work up, hydrolysis with 10% ethanolic potassium hydroxide solution and acidification, the E-diacid in 49% yield as well as a mixture of E- and Z-diacids. Cyclisation of the E-diacid with acetic anhydride gave E-fulgide (1) in 75% yield after purification.

On heating the fulgide (1) at 180°C in o-dichlorobenzene, it rearranged to the heliochromic compound (3), which was isolated in 50—60% yield from unreacted fulgide (1), its Z isomer and thianaphthene (13, Sheet 2) by recrystallisation from o-dichlorobenzene. Heliochromic compound (3) behaved similarly to the compound of Example 1 when irradiated with white light except that the coloured form (4) was purple.

Example 3

Scheme 1 R=Br (Sheet 3)

2-Bromo-4-acetylthiophen (15 g) was condensed with diethyl adamantylidenesuccinate (23 g) in toluene (100 $cm^3$) using sodium hydride (6 g) as condensing agent. Work up as described in Example 1, gave the E-fulgide of structure (1), as colourless needles from ethanol (7.5 g).

On irradiation at 366 nm fulgide (1) in toluene at 110°C rearranged quantitatively to the heliochromic compound (3), or alternatively, on heating the fulgide (2 g) at 180°C in o-dichlorobenzene for $\frac{1}{2}$ hour, it rearranged to the heliochromic compound in near quantitative yield. Heliochromic compound (3) reversibly produced a maroon coloured form on irradiation with white light.

Example 4

Scheme 1 R=$NO_2$ (Sheet 3)

E-fulgide (1 g), formula (1), R=H, was nitrated with concentrated nitric acid (0.43 g) in acetic anhydride (1.6 g). Work up gave Z-fulgide (0.3 g) and a mixture of E- and Z-fulgides. Irradiation at 366 nm of the Z-fulgide (0.3 g) in toluene at 20°C caused isomerisation to the E-fulgide (1), (R=$NO_2$) cyclisation to the 7,7a-DHBT (2), (R=$NO_2$) and rearrangement to the heliochromic compound (3), (R=$NO_2$). The coloured form (4) of the heliochromic compound was maroon.

Example 5

Scheme 2 R=H (Sheet 4)

3-Benzoylthiophen (5 g) was condensed with diethyl adamantylidenesuccinate (8 g) using potassium t-butoxide (3 g) in t-butanol (100 $cm^3$) as condensing agent. The mixture was boiled (1 h), t-butanol was distilled off under reduced pressure and the residue was acidified with hydrochloric acid and extracted with ether. Solvent was removed from the ether extract and the residual half-esters (formula 1A) was hydrolysed with 10% ethanolic potassium hydroxide. The diacids, obtained on acidification, were cyclised with acetic anhydride and gave a mixture of E and Z-fulgides.

The mixed fulgides (10 g) were heated ($\frac{3}{4}$ h) in tetralin (100 $cm^3$) at 208°C. Work up, gave the heliochromic compound (3), (R=H) (7.2 g) after crystallisation from ethanol. Compound (3) had a blue coloured form.

Example 6

Scheme 2 R—Ph (Sheet 4)

2-Phenyl-4-benzoylthiophen (13 g) was condensed with dimethyl adamantylidenesuccinate (14 g) in toluene (700 $cm^3$) using sodium hydride (5 g) as condensing agent. A mixture of E and Z fulgides was obtained in the manner described in Example 1.

On heating the mixed fulgides in tetralin, as described in Example 5, the heliochromic compound (3), (R=Ph) was obtained, which had a blue coloured form (4).

Example 7

Scheme 3 (Sheet 5)

3-Acetylthianaphthene (18 g) was condensed with diethyl adamantylidenesuccinate (29 g) in toluene (200 $cm^3$) using sodium hydride (11 g) as condensing agent. Work up including hydrolysis, acidification and cyclisation as described in Example 1, gave a mixture of E- and Z-fulgides which could be separated by fractional crystallisation from chloroform and petroleum (bg. 60—80°C).

On irradiation, at 366 nm, the E-fulgide (1) in toluene at 20°C, cyclised to form the structure (2) which then rearranged in near quantitative yield to give the heliochromic compound (3), obtained as yellow crystals after crystallisation from chloroform and petroleum (bp 60—80°C). Compound (3) reversibly formed a red compound (4) on irradiation with white light.

Example 8

Scheme 4 (Sheet 6)

3-Acetyl-4,5,6,7-tetrahydrothianaphthene (3 g) was condensed with dimethyl adamantylidenesuccinate (6 g) using potassium t-butoxide (3 g) in t-butanol (50 $cm^3$). The mixture was heated (2 h) at 80°C and worked up as described in Example 5 to give a mixture of E- and Z-fulgides, from which the Z-fulgide was separated by fractional crystallisation from ethanol, pale yellow prisms m.p. 185—187°C.

On irradiation at 366 nm, Z-fulgide isomerised to the E-fulgide which rearranged to the heliochromic compound (3), as described in Example 7. Compound (3) reversibly coloured blue on irradiation with U.V. light.

Example 9

Scheme 5, R=Me (Sheet 7)

2-Acetylthiophen (6 g) was condensed with diethyl adamantylidenesuccinate (16 g) in toluene (60 $cm^3$) in the presence of sodium hydride (5 g). Work up, as described in Example 2, gave E-fulgide (1) in 60% yield after crystallisation from ethanol.

On irradiation at 366 nm, fulgide (1) in toluene rearranged to the heliochromic compound (3) which was purified by chromatography on silica gel using toluene and petrol (1:1) as eluent, and obtained in 70% yield as yellow needles m.p. 197—198°C from dichloromethane and petrol. On exposure to white light, compound (3) underwent ring opening to form compound (4) which was red. The reverse reaction occurred in the dark.

Example 10

Scheme 5 R—Ph (Sheet 7)

2-Benzoylthiophen (10 g) was condensed with diethyl adamantylidenesuccinate (16 g) using potassium t-butoxide (6 g) in t-butanol (80 $cm^3$). Work up, as in Example 5, gave a mixture of pale orange E- and Z-fulgides.

On irradiation at 366 nm, the mixed fulgides in toluene at 55°C gave the heliochromic compound (3), (R—PH) in 70% yield, which was further purified by chromatography on silica gel using toluene and petrol as eluent, as in Example 9. The coloured form (4) was purple.

Example 11

Scheme 5

R=$CH_3O$ 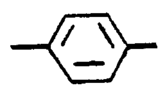 (Sheet 7)

2-p-Methoxybenzoylthiophen (2 g) was condensed with diethyl adamantylidenesuccinate (3 g) using potassium t-butoxide (2.5 g) in t-butanol (20 $cm^3$). Work up as in Examples 5 and 10, gave a mixture of bright yellow fulgides in 50% yield.

On irradiation at 366 nm, the mixed fulgides in toluene at 20°C gave the heliochromic compound (3), as in Example 10, whose coloured form was blue.

Example 12

Scheme 6 R=Me (Sheet 8)

3-Acetylfuran (2.5 g) was condensed with diethyl adamantylidenesuccinate (8 g) in toluene (100 $cm^3$) using sodium hydride (2 g) as condensing agent. Work up, gave the E-fulgide (1) (40 mg), as yellow needles, m.p. 160—161°C from chloroform and petroleum b.p. 60—80°C.

On heating (1 h) the E-fulgide at 180°C in deuterichloroform in a sealed tube, it rearranged to the

heliochromic compound (3), obtained as yellow needles, m.p. 210°C dec., from ether, which reversibly formed a maroon coloured compound (4) on irradiation with white light.

Example 13

Scheme 6 R=Ph (Sheet 8)

3-Benzoylfuran (4.5 g) was condensed with diethyl adamantylidenesuccinate (7.3 g) using potassium t-butoxide (8 g) in t-butanol (100 $cm^3$). Work up, as described in Example 5, gave a mixture of E- and Z-fulgides.

The mixed fulgides were converted into the heliochromic compound (3) as described in Example 5, purified by crystallisation from chloroform and petroleum (b.p. 60—80°C). The coloured form (4) of the heliochromic compound was blue.

Example 14

Scheme 7 (Sheet 9)

2-Benzoylbenzofuran (5 g) was condensed with dimethyl adamantylidenesuccinate (8 g) in toluene (100 $cm^3$) using sodium hydride (3 g) as condensing agent. Work up, as in Example 1, but using acetic anhydride (30 $cm^3$) instead of acetyl chloride, with diacid (3 g), gave E-fulgide (1) as yellow crystals from toluene and petroleum (b.p. 60—80°C), m.p. 257—259°C.

On irradiation at 366 nm, fulgide (1) in toluene at 110°C gave the heliochromic compound (3) which was purified by chromatography on silica gel using toluene and petroleum (1:1 mixture) as eluent. Compound (3) was reversibly converted to a red compound (4) on exposure to white light.

Example 15

Scheme 8 (Sheet 10)

E-fulgide (1, R=H in Scheme 1) (Sheet 3) (1 g), prepared as in Example 2, in acetone (20 $cm^3$) was treated with .880 ammonia (5 $cm^3$). After ½ h, acetone was removed and the residue carefully acidified with 5M hydrochloric acid and extracted with ether. The ethereal extract was dried over anhydrous magnesium sulphate, filtered and ether removed. The resulting acid amide was converted into the methyl ester amide, by reaction with diazomethane in ether and this ester amide was boiled (0.5 h) with methanolic solution of sodium methoxide (freshly prepared by dissolving sodium (0.5 g) in methanol (100 $cm^3$). Work up, gave the E-fulgide (1) (0.65 g), crystals from ethanol.

On irradiation at 366 nm, fulgimide (1) in toluene at 20°C rearranged to give the heliochromic compound (3). Compound (3) was reversibly convertible to a maroon coloured compound (4) on exposure to white light.

Routes to the corresponding heliochromic imides include rearrangement of the corresponding fulgimides by heating in solvent in an analogous manner to the corresponding fulgides. Alternatively, the heliochromic anhydride could be converted to its corresponding imide by reaction with ammonia or an aliphatic or aromatic amine (see as illustrated in Example 15).

The following Examples also illustrate the preparation of fulgimides and their conversion into heliochromic compounds.

Example 16

Scheme 9 (Sheet 11)

Benzothiophen (1) in ether was treated with an ethereal solution of butyl lithium and the resulting 2-lithiobenzothiophen (2) was reacted with dimethylacetamide (3). Work up gave 2-acetylbenzothiophen (4), as colourless crystals.

Ketone (4) was condensed with dimethyl adamantylidenesuccinate in the presence of sodium hydride in toluene. The resulting half-ester was hydrolysed with ethanolic potassium hydroxide and acidified with hydrochloric acid to yield diacid (5), which was cyclised to E-fulgide (6), using acetic anhydride.

E-fulgide (6), obtained in bright yellow crystals, was dissolved in acetone and treated with conc.ammonia. The resulting succinamic acid (7, R=H) was converted into the corresponding methyl ester (7, R=Me) using ethereal diazomethane and hence into E-fulgimide (8) by reaction with freshly prepared sodium methoxide in methanol. To monitor the course of reaction by n.m.r. spectroscopy, E-fulgimide (8) was irradiated at 366 nm for 3 days in $CDCl_3$. It photocyclised to photochrome (9) which, in turn, underwent a 1,5-hydrogen shift to yield heliochromic imide (10). Imide (10) can be reversibly converted into an orange-coloured form (11) by U.V. irradiation in toluene. On removal of the U.V. stimulation, the compound (11) reverted to its colourless form (10).

Example 17

(Scheme 10) (Sheet 12)

Fulgide (1), obtained by the procedure described in Example 8, can be reacted with ammonia in acetone to form the succinamic acid (2) (R=H), followed by treatment with etheral diazomethane to form the corresponding methyl ester. The ester compound (2) (R=Me), can be treated with freshly prepared sodium methoxide in methanol. The resulting fulgimide (3) was photochromic, showing a colourless to red colour change on irradiation at 366 nm, reversed by white light.

On irradiation at 366 nm in $CDCl_3$ for 3 days, fulgimide (3) is converted into heliochromic compound (5). In sunlight, compound (5) undergoes a colourless to orange colour change to compound (6) and thermally fades to the colourless forms.

Example 18

1. Preparation of 4-acetyl-2-phenyl thiophene (Sheet 13)

Phenylacetaldehyde (1) (30 g) was reacted with sulphur (6 g) and ethyl cyanoacetate (19 g) in the presence of morpholine (17.5 g) and ethanol (50 ccs) to give the crystalline amino ester (2) (11.6 g).

The amino ester (2) was converted to the diazonium salt and reduced by boiling in ethanol containing finely divided copper to give the pure ester (3). Hydrolysis of the ester with aqueous ethanolic KOH followed by acidification with 5M HCl, gave the acid (4) in approximately 70% yield. The acid was converted to the corresponding acid chloride (5) by reaction with $SOCl_2$.

The acid chloride (5) was reacted with magnesium diethyl malonate to yield the compound (5A) as an intermediate and the ketone (6) as the final product. The resulting ketone can be converted to the corresponding heliochrome as described in Example 1.

Example 19

4-acetyl-2-(3',4'-dimethoxyphenyl) thiophene was prepared as described in Example 18 using 3,4-dimethoxyphenyl acetaldehyde as the starting material instead of phenyl acetaldehyde. The resulting methyl ketone (75 g) (Compound (6) Sheet 13 — phenyl replaced by 3,4-dimethoxy phenyl) was condensed with dimethyl succinate (140 g) in the presence of sodium hydride (100 g) in toluene, followed by hydrolysis to form the diacid. Cyclisation with hot acetic anhydride gave the E-fulgide which was purified by chromatography on silica gel. On irradiation at 366 nm in toluene, the fulgide was converted into the heliochromic compound of formula (3), Sheet 4, R = 3,4-dimethoxy phenyl. On exposure to sunlight or to a flash gun the compound turned deep blue.

The heliochromic compounds described herein are solvatochromic i.e. their absorption spectra depends upon the solvent. Thus, in the case of photoreactive lenses, the nature of the colour change can be varied by selecting the plastics material forming the lenses or the coating thereon.

Investigations of the colour fade rate after removal of the U.V. light stimulation, shows that this substantially follows first order kinetics. This is in contrast with the fade rates of known photoreactive lenses based on silver dispersions, whose fade rate decreases rapidly with time so that a very considerable time delay is required before the colour fade is substantially complete.

The heliochromic compounds of this invention can be added to a polymerisation mixture from which plastics lenses are intended to be produced. For example, in one test methyl methacrylate (53 cm) containing the heliochrome of formula 9 (Sheet 1) (0.15 g) and benzoyl peroxide (0.15 g) was polymerised by heating at 100°C for 30 minutes. The resulting polymer was found to be heliochromic, which indicates that the heliochromes are partially resistant to peroxide-induced degradation.

The discovery of photochromic materials which can be used to render plastic lenses photoreactive is a particularly valuable one since, until the present invention, it has not been possible to produce photoreactive plastic lenses which are capable of undergoing more than a small number of colour change cycles. It is possible in principle to incorporate or coat any type of plastics (or glass) lens but generally it is envisaged that the compounds of this invention will be used to render photoreactive the usual types of plastic lenses; these are essentially polycarbonate and alkyl acrylate and methacrylate lenses. Methods of manufacturing plastic lenses are described for example in U.S. Patent Nos. 3,944,637, 2,542,386 and 3,404,861, the disclosure of which is incorporated herein by reference.

The most commonly used material for plastic lenses is diethylene glycol bis (allyl carbonate) usually known as CR-39 (CR-39 is a trade mark of P.P.G. Ltd.). The heliochromic compound may be mixed or combined into the CR-39 catalysed liquid monomer, degassed and poured into glass moulds and cured, e.g. as described in U.S. Patent No. 2,542,286, the disclosure of which is incorporated herein by reference for further details of the procedure for moulding or casting plastic lenses.

A variety of techniques are available for preparing heliochromic plastics articles in accordance with the teaching of this invention. The heliochromic compounds need not be incorporated directly into or coated onto the plastics articles, but can be employed in the form of fulgide or fulgimide precursors and converted *in situ*, during or after the incorporation or coating step, into the corresponding heliochromic compound, e.g. by heating at U.V. irradiation. Conversion *in situ* provides an advantageous route to the manufacture of heliochromic plastics articles, such as photoreactive lenses.

It has been found, for example, that an effective amount of precursor is incorporated into a plastics article, such as a lens by contacting in substantially oxygen-free conditions at least one surface of the lens with a melt or solution of the fulgide or fulgimide, which is the precursor of the desired heliochromic compound. Preferably the treatment is effected at elevated temperature, e.g. about 180°C, in order to increase the rate of diffusion of the fulgide or fulgimide into the plastics article. Although at this temperature, the fulgides and fulgimides undergo conversion to the corresponding heliochromic compound, it is believed that the fulgide or fulgimide diffuses preferentially into the plastics article. Possibly this is because the heliochromic compounds tend to have higher melting points than their

precursors. In any event, crystals of heliochromic compound are observable in the precursor melts used to treat the preformed plastics articles.

While the precursors may be employed in the form of melts and are generally quite stable in molten condition at a temperature of about 180°C for a few hours, precursor can also be imbibed into the plastics articles from a solution. Solvents employed should, of course, be inert and are preferably high boiling, e.g. boiling at temperatures above 200°C so that the imbibation from a solution can be carried out at elevated temperature in order to increase the rate of diffusion.

In the case of solvents, advantage may be taken of a technique employed in disperse dyeing of textiles or fibres where a solvent is selected which possesses a lesser affinity for the solute (in this case the precursor rather than a dyestuff) than does the material to be treated (in this case the plastics article). As a result the precursor is effectively partitioned between solution in the solvent and solid solution in the plastic and thus diffuses into the plastics article. Generally high boiling points solvents such as fluorinated hydrocarbons or silicone oils are suitable from this viewpoint when imbibing the precursor into CR39 plastics articles. Usually a saturated solution of the precursor in the selected solvent is used, which would not normally exceed a concentration of a few per cent of the precursor. The following Examples illustrate this aspect of the invention:

Example 20

A quantity of the fulgide prepared in Example 2 (Sheet 3, formula (1), R=H) was melted in a concave depression in a hot plate heated to a temperature of 180°C. The surface of a cured CR-39 plastics lens was treated with the molten precursor by laying its convex surface (whose curvature roughly corresponded with the concavity of the depression) in the depression in the hot plate. In this way the surface of the lens was maintained in contact with the molten precursor for 2 hours.

After this treatment the lens was exposed to radiation from an AM 2 lamp at 25°C and was found to be heliochromic, darkening to 5.5% transmission. On removal of the stimulation from the lamp, the time required to fade to half the initial optical density was 32 seconds. The fatigue life (T1/2) was 2000 minutes. T1/2 is the time taken for the induced optical density to decay to half its initial value when exposed continuously to Air Mass 2 radiation.

Example 21

A saturated solution was prepared of the same fulgide as used in Example 20 in the fluorocarbon solvent sold by Minnesota Mining and Manufacturing Company (3M) under the trade name FC 70. This solvent had a boiling point of 217°C. The resulting solution was heated to 180°C and a 2 mm thick coplanar CR39 lens was immersed in the solution for one hour while maintaining the temperature at 180°C. After removal from the solvent, the lens was found to be heliochromic and darkened to an induced optical density of 0.7 when irradiated with an AM 2 lamp. Thermal reverse was very rapid at ambient temperature when removing the white light irradiation.

Reference is made herein to our European Patent Application No. 84306032.8 filed concurrently herewith, inventors Baskerville et al, (Attorney's Reference No. DCW/P.201), which describes preparation of heliochromic plastics articles, such as photoreactive lenses, by incorporating precursors of the heliochromic compounds of this invention into plastic moulding and casting compositions, and forming the heliochromic compound *in situ* in the plastic article during the moulding or casting step or in an after treatment thereof. The disclosure of the Baskerville application is specifically incorporated herein by reference.

**Claims for the Contracting States: BE CH DE FR IT LI LU NL SE**

1. A compound of the following general formula:

(I)

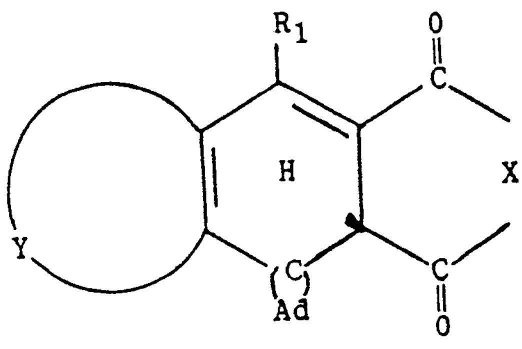

Ad⌒C< represents an adamantylidene or a substituted adamantylidene group; $R_1$ represents hydrogen, alkyl, aryl or aralkyl; X represents oxygen or $>NR_2$, in which $R_2$ is hydrogen, aryl, alkyl or aralkyl and

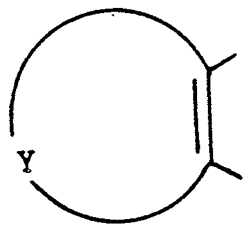

represents an aromatic group, an unsaturated heterocyclic group or a benzannelated heterocyclic group.

2. A heliochromic compound having the following general formula:

(I)

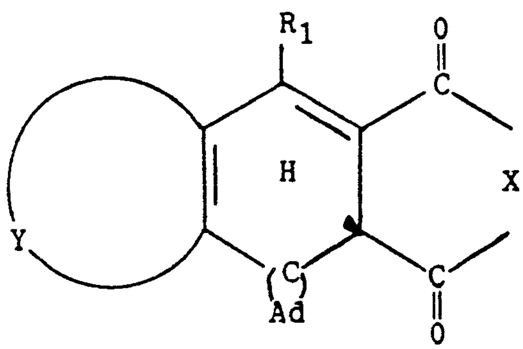

wherein $R_1$ X and Ad⌒C> have the same significance as in Claim 1, and

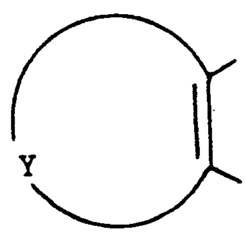

is derived from a 2,5-; 2,4- or 3,5-dimethoxyphenyl group or from a 2- or 3- furyl or a 2- or 3- thienyl group, which may be benzannelated.

3. A compound according to claim 2 in which the furyl or thienyl group is deuterated or substituted with one or more nitro, aryl, or halogen groups.

4. A compound according to claim 2 in which $R_1$ is a lower alkyl or aryl group.

5. A compound according to claim 1 in which the ring represented by

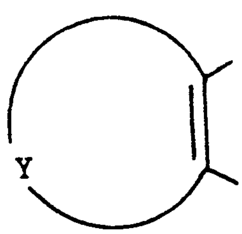

is a 5-membered heterocyclic or benzannelated heterocyclic ring containing oxygen or sulphur and at least one double bond.

6. A method of preparing a compound having the general formula set forth in claim 1 which comprises subjecting a compound of the following general formula (IV) to heating in a solvent to a temperature above 100°C or to concomitant U.V. radiation and heating or to treatment with a Lewis acid and recovering the rearrangement product:

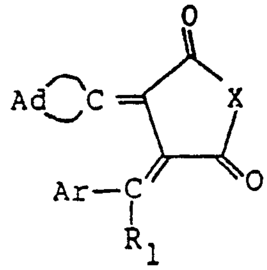

(IV)

wherein:
Ad⌒C< represents an adamantylidene or a substituted adamantylidene group; $R_1$ represents hydrogen, alkyl, aryl or aralkyl; and Ar represents a benzene ring or a 5-membered heterocyclic or benzannelated heterocyclic ring containing oxygen or sulphur and X represents oxygen or $>NR_2$, wherein $R_2$ is hydrogen, aryl, alkyl or aralkyl.

7. A method according to claim 6 in which Ar represents a benzene ring containing 2,5-; 2,4- or 3,5-dimethoxy substituents or a 2- or 3- furyl or thienyl group.

8. A method according to claim 7 in which the furyl or thienyl group is benzannelated or substituted with lower alkyl or phenyl.

9. A photoreactive ophthalmic or plano lens having a heliochromic compound coated on a surface thereof or laminated or incorporated in the material of the lens, the heliochromic compound having the property of reversibly forming a coloured structural form on irradiation with U.V. light and returning to a colourless or paler structural form at normal ambient temperature in the absence of U.V. stimulation, the coloured form having the following structure (a)

(a)

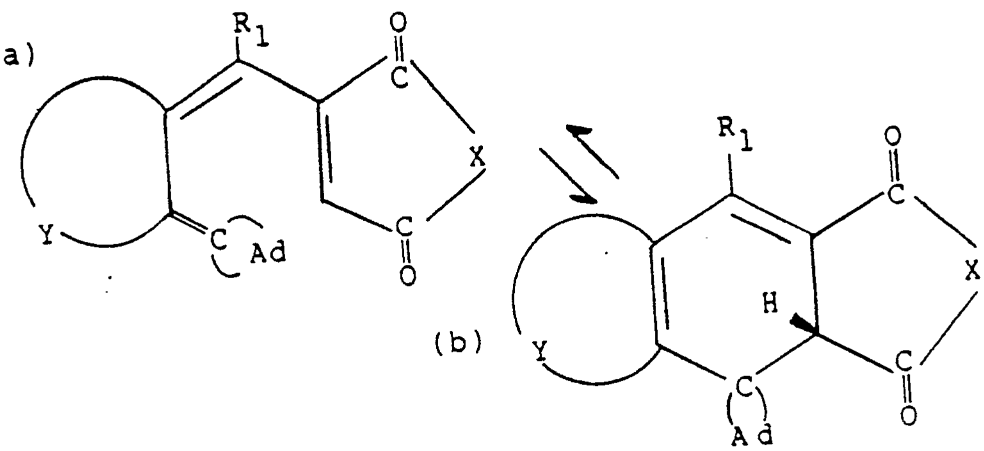

(b)

and being capable of conversion to its colourless or paler form (b) by formation of a central 6-membered ring, wherein

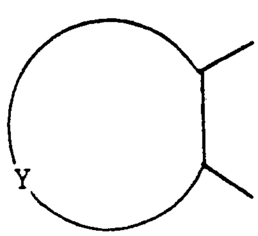

, $R_1$, X

and Ad⌒C< have the same significance as in claim 1.

10. A photoreactive ophthalmic or plano lens having a heliochromic compound coated on a surface thereof or laminated or incorporated in the material of the lens, the heliochromic compound having the following general formula:

(I')

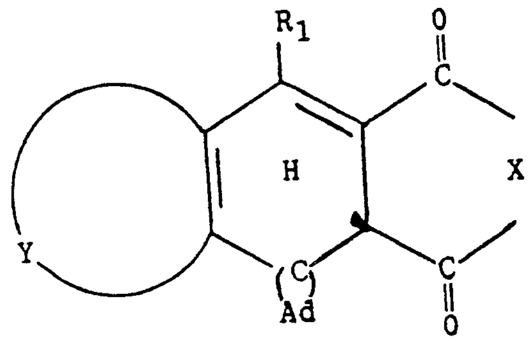

wherein $R_1$, X and Ad⌒C< have the same significance as in claim 1, and

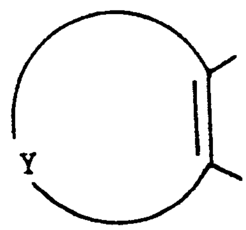

is derived from a 2,5; 2,4- or 3,5-dimethoxyphenyl group; from a 2- or 3- furyl or from a 2- or 3- thienyl group, which may be benzannelated or substituted.

11. A photoreactive lens according to claim 10 wherein the lens is formed from a polycarbonate or a polyalkyl methacrylate or acrylate.

12. A photoreactive ophthalmic or plano lens which comprises a blend of heliochromic compounds, as claimed in claim 1 or claim 2, coated on a surface thereof or laminated or incorporated in the material of the lens.

**Claims for the Contracting State: AT**

1. A method of preparing a compound having the general formula:

(I)

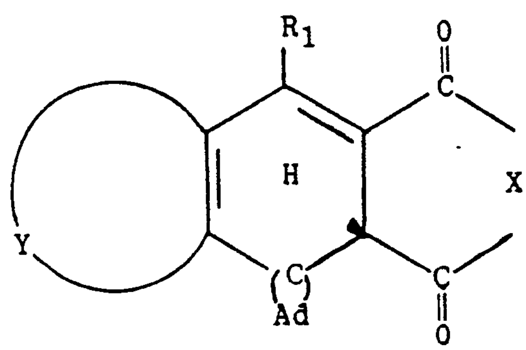

which comprises subjecting a compound of the following general formula (IV) to heating in a solvent to a temperature above 100°C or to concomittant U.V. radiation and heating or to treatment with a Lewis acid and recovering the rearrangement product:

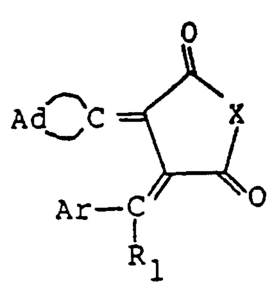

(IV)

wherein Ad⁀C< represents an adamantylidene group; $R_1$ represents hydrogen, alkyl, aryl or aralkyl; and Ar represents a benzene ring or a 5-membered heterocyclic or benzannelated heterocyclic ring containing oxygen or sulphur and X represents oxygen or $>NR_2$, wherein $R_2$ is hydrogen, aryl, alkyl or aralkyl.

2. A method according to claim 1 in which Ar represents a benzene ring containing 2,5-; 2,4- or 3,5-dimethoxy substituent or a 2- or 3- furano or thiopheno group.

3. A method according to claim 2 in which the furano or thiopheno group is benzannelated or substituted with lower alkyl or phenyl.

4. A photoreactive ophthalmic or plano lens having a heliochromic compound coated on a surface thereof or laminated or incorporated in the material of the lens, the heliochromic compound having the property of reversibly forming a coloured structural form on irradiation with U.V. light and returning to a colourless or paler structural form at normal ambient temperature in the absence of U.V. stimulation, the coloured form having the following structure (a)

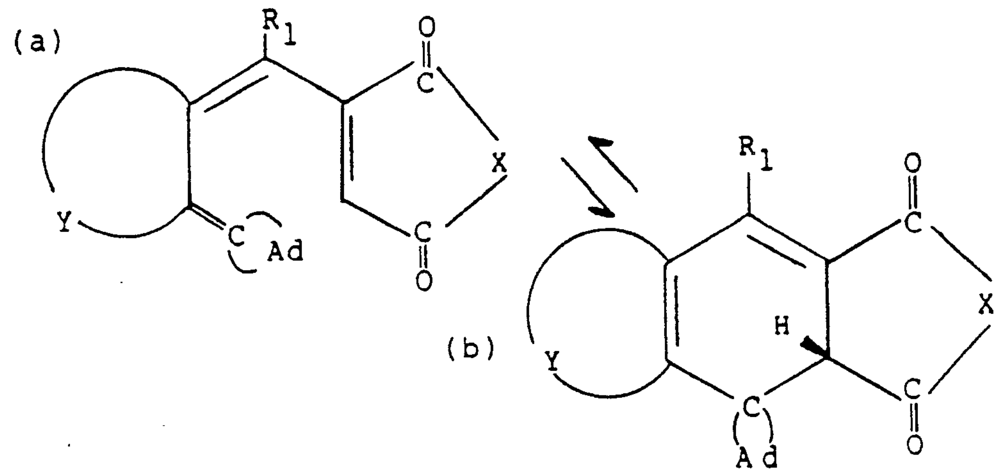

and being capable of conversion to its colourless or paler form (b) by formation of a central 6-membered ring, wherein Ad⁀C< represents an adamantylidene group, $R_1$ represents hydrogen, alkyl, aryl or aralkyl; X represents oxygen or $>NR_2$ in which $R_2$ is hydrogen, aryl, alkyl or aralkyl and

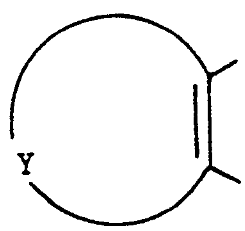

represents an aromatic group, an unsaturated heterocyclic group or a benzannelated heterocyclic group.

5. A photochromic ophthalmic or plano lens having a heliochromic compound coated on a surface thereof or laminated or incorporated in the material of the lens, the heliochromic compound having the following general formula:

(I) 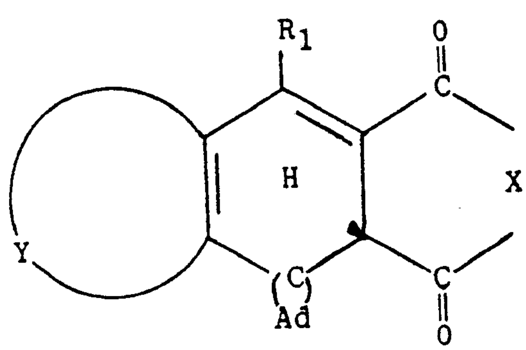

wherein $R_1$, X and Ad⌣C< have the same same significance as in claim 1, and

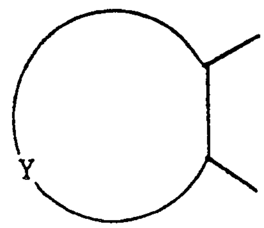

is derived from a 2,5-; 2,4- or 3,5-dimethoxybenzo group or from a 2- or 3- furano or a 2- or 3- thiopheno group.

6. A photoreactive lens according to claim 5 wherein the lens is formed from a polycarbonate or a polyalkyl methacrylate or acrylate.

**Patentansprüche für die Vertragsstaaten: BE CH DE FR IT LI LU NL SE**

1. Eine Verbindung der allgemeinen Formel

(I)

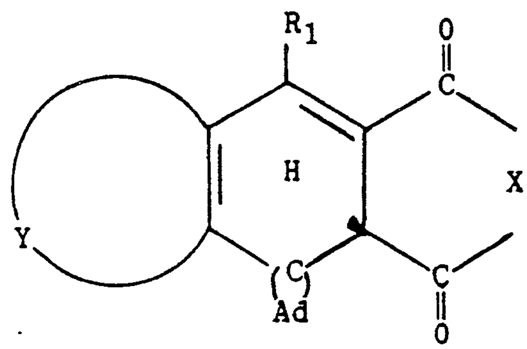

wobei Ad⌣C< eine Adamantylidengruppe oder eine substuierte Adamantylidengruppe bedeutet, $R_1$ Wasserstoff, Alkyl-, Aryl- oder Aralkyl-Gruppen, X Sauerstoff oder >$NR_2$, wobei $R_2$ Wasserstoff, Aryl-, Alkyl- oder Aralkyl-Gruppen bedeutet, und

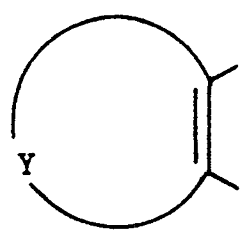

eine aromatische Gruppe ist oder eine ungesättigte heterozyklische Gruppe oder eine um einen Benzolring erweiterte (benzannelierte) heterozyklische Gruppe.

2. Eine heliochromische Verbindung der allgemeinen Formel

(I)

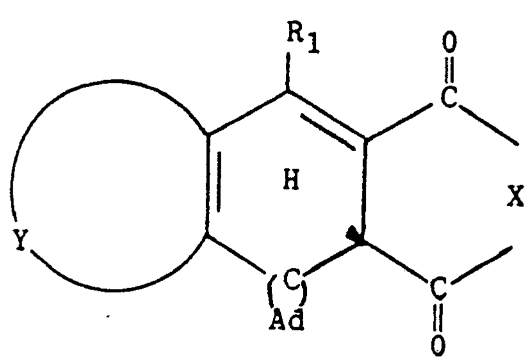

dadurch gekennzeichnet, daß $R_1$, X und Ad⌢C< eine Bedeutung gemäß Anspruch 1 haben, und

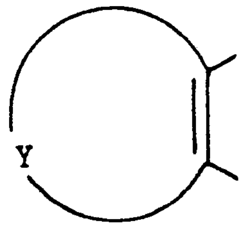

sich ableitet von einer 2,5-; 2,4- oder 3,5-Dimethoxyphenyl-Gruppe oder von einer 2- oder 3-Furyl-Gruppe oder einer 2- oder 3-Thienyl-Gruppe, welche um einen Benzolring erweitert (benzanneliert) sein können.

3. Verbindung gemäß Anspruch 2, dadurch gekennzeichnet, daß die Furyl- oder Thienyl-Gruppe deuteriert ist oder mit einem oder mehreren Nitro-, Aryl- oder Halogen-Resten substituiert ist.

4. Verbindung nach Anspruch 2, dadurch gekennzeichnet, daß $R_1$ eine niedere Alkyl- oder Aryl-Gruppe ist.

5. Verbindung gemäß Anspruch 1, dadurch gekennzeichnet, daß der Ring gemäß der Formel

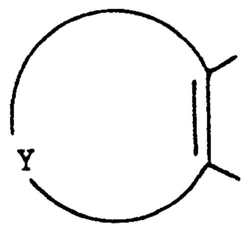

ein fünfgliederiger heterozyklischer oder um einen Benzolring erweiterter heterozyklischer Ring ist, der Sauerstoff oder Schwefel und mindestens eine Doppelbindung aufweist.

6. Verfahren zur Herstellung einer Verbindung der allgemeinen Formel gemäß Anspruch 1, dadurch gekennzeichnet, daß eine Verbindung der allgemeinen Formel (IV)

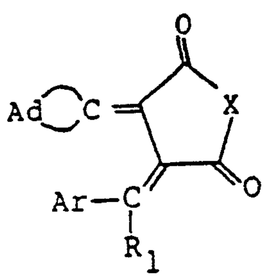

(IV)

in einem Lösungsmittel auf Temperaturen über 100°C erhitzt wird oder zusätzlich einer UV-Bestrahlung oder der Behandlung mit einer Lewis-Säure ausgesetzt wird, unter Aufarbeitung des Umlagerungsproduktes, wobei in obiger Formel Ad⌢C< eine Adamantyliden- oder eine substituierte Adamantyliden-Gruppe bedeutet, $R_1$ Wasserstoff, Alkyl-, Aryl-, oder Aralkyl-Gruppen und Ar einen Benzolring oder einen fünfgliedrigen heterozyklischen Ring oder einen um einen Benzolring erweiterten (benzannilierten) heterozyklischen Ring, mit Sauerstoff oder Schwefel, und wobei X Sauerstoff oder $>NR_2$ bedeutet, wobei $R_2$ Wasserstoff, Aryl-, Alkyl- oder Aralkyl-Gruppen sein können.

7. Verfahren nach Anspruch 6, dadurch gekennzeichnet, daß Ar eine Benzolring bedeutet mit 2,5-; 2,4- oder 3,5-Dimethoxysubstituenten oder eine 2- oder 3-Furyl- oder Thienyl-Gruppe.

8. Verfahren nach Anspruch 7, dadurch gekennzeichnet, daß die Furyl- oder Thienyl-Gruppe um einen Benzolring erweitert oder substituiert ist mit einer niedrigen Alkyl- oder einer Phenyl-Gruppe.

9. Photoreaktives Brillenglas oder Planlinse mit einer heliochromischen Verbindung auf deren Oberfläche als Coating oder Laminat oder aber in das Glas- bzw. Linsenmaterial inkorporiert, wobei die heliochromische Verbindung die Eigenschaft aufweist, bei der Bestrahlung mit UV-Licht reversibel eine gefärbte Struktur anzunehmen und zu einer farbschwächeren oder farblosen Struktur zurückzukehren bei normaler Umgebungstemperatur und bei Abwesenheit einer UV-Stimulation, wobei die gefärbte Form die folgende Struktur (a) aufweist und fähig ist, sich in die farblose oder farbschwächere Form (b) umzulagern unter Bildung eines zentralen sechsgliedrigen Ringes

(a)

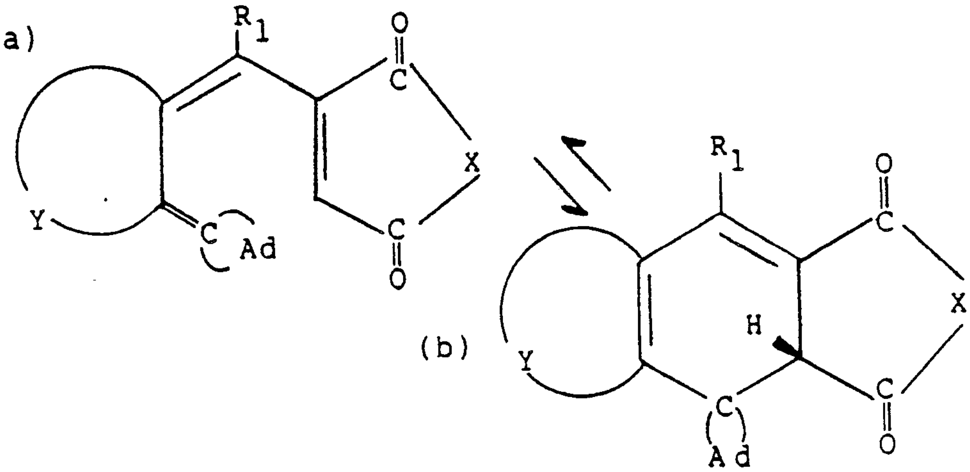

(b)

wobei die y enthaltende Ringgruppe, $R_1$, X und Ad⊃C< dieselbe Bedeutung haben wie in Anspruch 1.

10. Photoreaktives Brillenglas oder Planlinse mit einer heliochemischen Verbindung auf deren Oberfläche als Coating oder Laminat oder aber in das Glas- bzw. Linsenmaterial inkorporiert, wobei die heliochromische Verbindung die allgemeine Formel

(I)

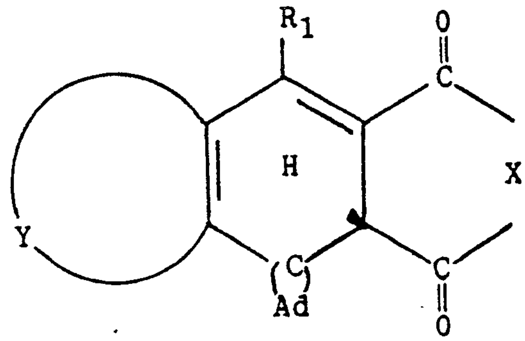

aufweist, wobei $R_1$, X und Ad⊃C< dieselbe Bedeutung haben wie in Anspruch 1 und

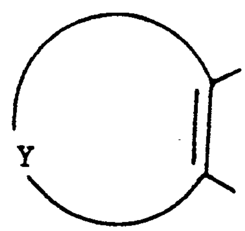

sich ableitet von einer 2,5-; 2,4- oder 3,5-Dimethoxyphenyl-Gruppe; oder von einer 2- oder 3-Furyl-Gruppe oder von einer 2- oder 3-Thienyl-Gruppe, welche Substituenten aufweisen oder um einen Benzolring erweitert (benzanneliert) sein können.

11. Photoreaktives Brillenglas oder Planlinse gemäß Anspruch 10, dadurch gekennzeichnet, daß die aus Polykarbonat, Polyalkylmethacrylat oder einem Acrylat bestehen.

12. Photoreaktives Brillenglas oder Planlinse, dadurch gekennzeichnet, daß sie ein Gemisch von heliochromischen Verbindungen gemäß Anspruch 1 oder 2 inkorporiert enthalten, oder damit in Form eines Coatings oder Laminats beschichtet sind.

**Patentansprüche für den Vertragsstaat: AT**

1. Verfahren zur Herstellung einer Verbindung der allgemeinen Formel

(I)

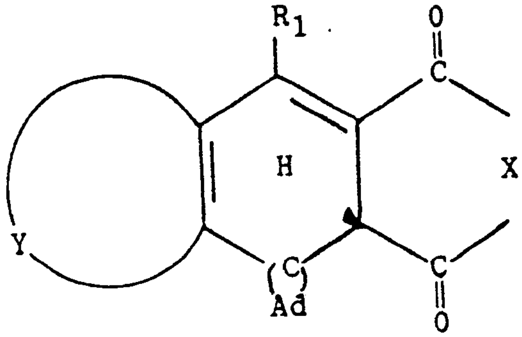

dadurch gekennzeichnet, daß eine Verbindung der allgemeinen Formel (IV)

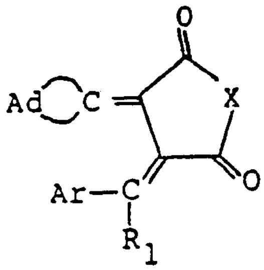

(IV)

in einem Lösungsmittel auf Temperaturen über 100°C erhitzt wird oder zusächst einer UV-Bestrahlung oder der Behandlung mit einer Lewis-Säure ausgesetzt wird, unter Aufarbeitung des Umlagerungsproduktes, wobei in obiger Formel Ad⌒C< eine Adamantyliden- oder eine substituierte Adamantyliden-Gruppe bedeutet, $R_1$ Wasserstoff, Alkyl-, Aryl-, oder Aralkyl-Gruppen und Ar einen Benzolring oder einen fünfgliedrigen heterozyklischen Ring oder einem um einen Benzolring erweiterten (benzannelierten) heterozyklischen Ring, mit Sauerstoff oder Schwefel, und wobei X Sauerstoff oder $>NR_2$ bedeutet, wobei $R_2$ für Wasserstoff, Aryl-, Alkyl- oder Aralkyl-Gruppen steht.

2. Verfahren nach Anspruch 1, dadurch gekennzeichnet, daß Ar eine Benzolring bedeutet mit 2,5-; 2,4- oder 3,5-Dimethoxy-Substituenten oder eine 2- oder 3-Furan- oder Thiophen-Gruppe.

3. Verfahren nach Anspruch 2, dadurch gekennzeichnet, daß die Furan- oder Thiophen-Gruppe um einen Benzolring erweitert (benzanneliert) oder substituiert ist mit einer niedrigen Alkyl- oder einer Phenyl-Gruppe.

4. Photoreaktives Brillenglas oder Planlinse mit einer heliochromischen Verbindung auf deren Oberfläche als Coating oder Laminat oder aber in das Glas- bzw. Linsenmaterial inkorporiert, wobei die heliochromische Verbindung die Eigenschaft aufweist, bei der Bestrahlung mit UV-Licht reversibel eine gefärbte Struktur anzunehmen und zu einer farbschwächeren oder farblossen Struktur zurückzukehren bei normaler Umgebungstemperatur und bei Abwesenheit einer UV-Stimulation, wobei die gefärbte Form die folgende Struktur (a) aufweist und fähig ist, sich in die farblose oder farbschwächere Form (b) umzulagern unter Bildung eines zentralen sechsgliedrigen Ringes

(a)

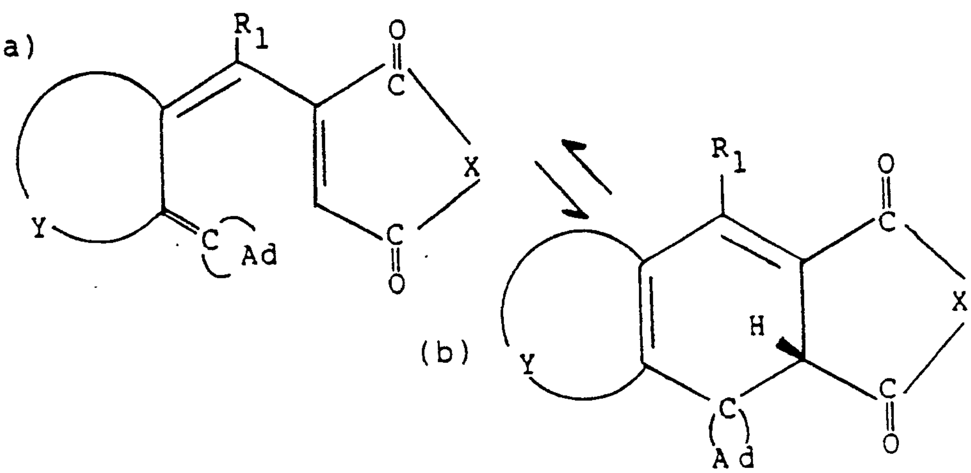

(b)

wobei Ad⁀C< eine Adamantyliden-Gruppe bedeutet, $R_1$ Wasserstoff, eine Aryl-, Alkyl- oder Aralkyl-Gruppe, X Sauerstoff oder $>NR_2$, wobei $R_2$ Wasserstoff oder Aryl-, Alkyl- oder Aralkyl-Gruppen sind, und wobei die Y enthaltende Ringgruppe eine aromatische Gruppe bedeutet oder eine ungesättigte heterozyklische Gruppe oder eine um einen Benzolring erweiterte (benzannelierte) heterozyklische Gruppe.

5. Photoreaktives Brillenglas oder Planlinse mit einer heliochromischen Verbindung auf deren Oberfläche als Coating oder Laminat oder aber in das Glas- bzw. Linsenmaterial inkorporiert, wobei die heliochromische Verbindung die allgemeine Formel

(I) 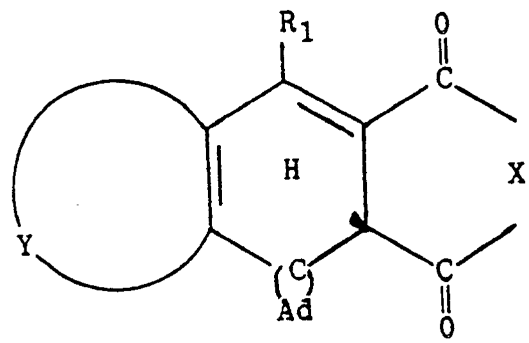

aufweist, wobei $R_1$, X und Ad⁀C< dieselbe Bedeutung haben wie in Anspruch 1 und

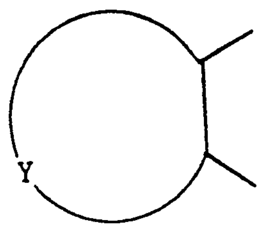

sich ableitet von einer 2,5-; 2,4- oder 3,5-Dimethoxybenzo-Gruppe oder von einer 2- oder 3-Furan- oder einer 2- oder 3-Thiophen-Gruppe.

6. Photoreaktives Brillenglas oder Planlinse nach Anspruch 5, dadurch gekennzeichnet, daß sie aus Polykarbonat, Polyalkylmethacrylat oder Acrylat bestehen.

**Revendications pour les Etats contractants: BE CH DE FR GB IT LI LU NL SE**

1. Composé de formule générale suivante:

(I) 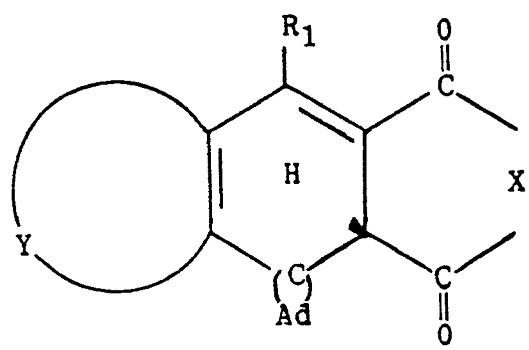

Ad⁀C< représente un adamantylidène ou un groupe adamantylidène substitué;
$R_1$ représente hydrogène, alkyle, aryle ou aralkyle
X représente oxygène ou $>NR_2$, dans lequel $R_2$ est hydrogène, aryle, alkyle ou aralkyle et

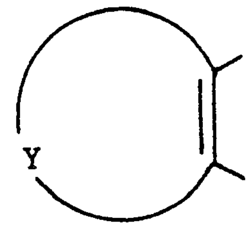

représente un groupe aromatique, un groupe hétérocyclique insaturé ou un groupe hétérocyclique à anneaux benzéniques.

2. Composé héliochromique ayant la formule générale suivante:

(I) 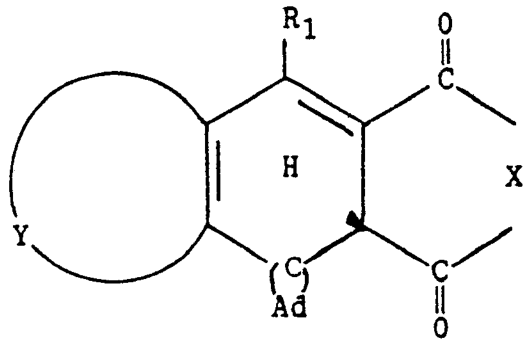

dans laquelle $R_1$ X et Ad⌒C< ont la même signification que dans la revendication 1, et

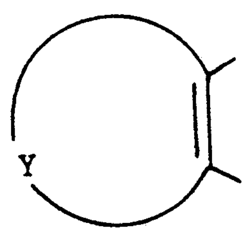

est dérivé d'un groupe 2,5-; 2,4- ou 3,5-diméthoxyphényle ou d'un groupe 2- ou 3- furyle ou d'un groupe 2- ou 3-thiényle, qui peut être à anneaux benzéniques.

3. Composé selon la revendication 2 dans lequel le groupe furyle ou thiényle est deutérié ou substitué avec un ou plusieurs groupes nitro, aryle ou halogène.

4. Composé selon la revendication 2 dans lequel $R_1$ est un groupe alkyle ou aryle inférieur.

5. Composé selon la revendication 1, dans lequel l'anneau représenté par

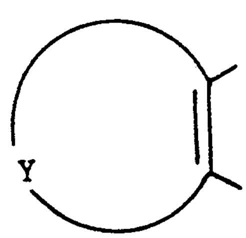

est un anneau hétérocyclique à 5 membres ou un anneau hétérocyclique à anneau benzénique contenant oxygène ou soufre et au moins une double liaison.

6. Procédé de préparation d'un composé ayant la formule gènerale énoncée dans la revendication 1, comprenant le fait de soumettre un composé de formule générale (IV) suivante à un chauffage dans un solvant jusqu'à une température dépassant 100°C ou à un rayonnement ultraviolet et à un chauffage concomitants ou à un traitement par un acide de Lewis et le fait de récupérer le produit de réarrangement:

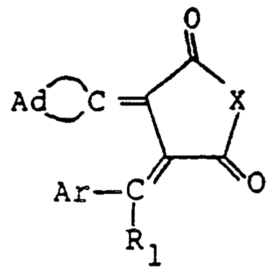

(IV)

dans lequel Ad⌢C< représente un adamantylidène ou un groupe adamantylidène substitué, $R_1$ représente hydrogène, alkyle, aryle ou aralkyle, et Ar représente un anneau benzène ou un anneau hétérocycliques à 5 membres ou un anneau hétérocyclique à anneau benzénique contenant oxygène ou soufre et X représente oxygène ou $>NR_2$, dans lequel $R_2$ est hydrogène, aryle, alkyle ou aralkyle.

7. Procédé selon la revendication 6 dans lequel Ar représente un anneau benzène contenant des substituants 2,5-; 2,4- ou 3,5-diméthoxy ou un groupe 2- ou 3- furyle ou thiényle.

8. Procédé selon la revendication 7 dans lequel le groupe furyle ou thiényle a un anneau benzénique ou est substitué avec un alkyle ou phényle inférieur.

9. Lentille photoréactive, ophthalmique ou plane, ayant un composé héliochromique en revêtement sur une de ses surfaces ou laminé ou incorporé dans la matière de la lentille, le composé héliochromique ayant la propriété de former de manière réversible une forme structurelle colorée en étant irradié par la lumière ultra-violette et de revenir en une forme structurelle incolore ou plus pâle à la température ambiante normale en l'absence de stimulation ultra-violette, la forme colorée ayant la structure (a) suivante:

(a)

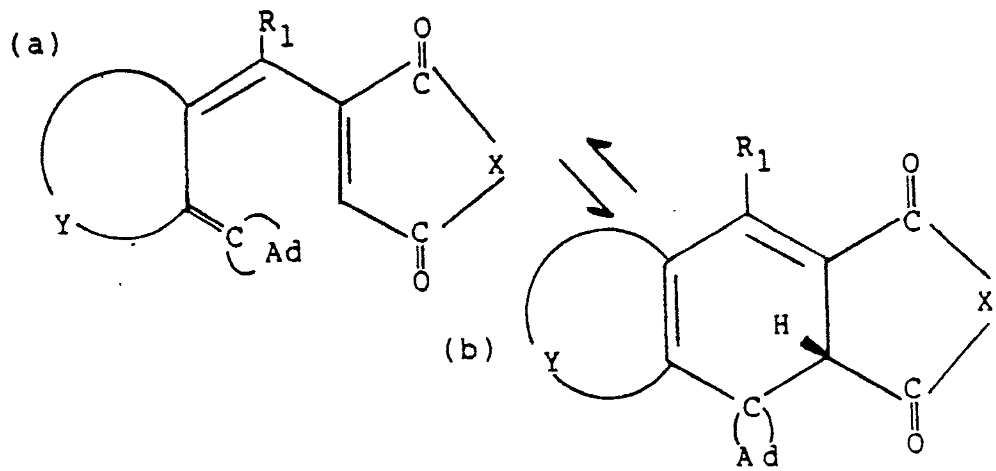

(b)

et étant capable de conversion vers sa forme (b) incolore ou plus pâle par formation d'un anneau central à 6 membres, dans lequel

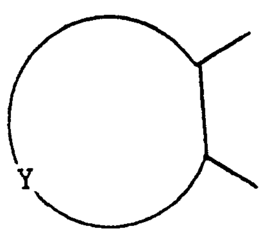

, $R_1$, X

et Ad⌢C< on la même signification que dans la revendication 1.

10. Lentille photoréactive, ophthalmique ou plane ayant un composé héliochromique en revêtement sur une de ses surfaces, ou laminé ou incorporé dans la matière de la lentille, le composé héliochromique ayant la formule générale suivante:

(I) 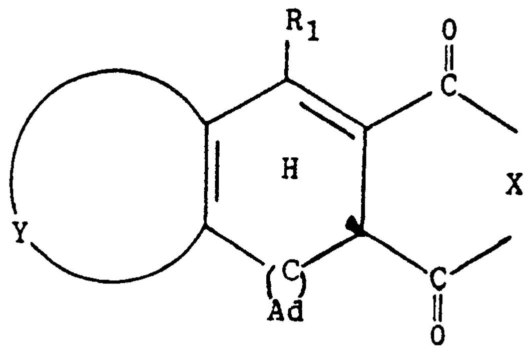

dans laquelle $R_1$, X et Ad⌒C<ont la même signification que dans la revendication 1, et

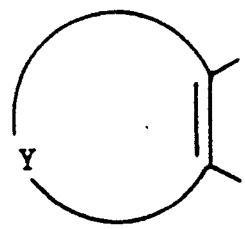

est dérivé d'un group 2,5, 2,4, ou 3,5- diméthoxyphényle; d'un groupe 2- ou 3-furyle ou d'un groupe 2- ou 3-thiényle, qui peut être à anneau benzénique ou être substitué.

11. Lentille photoréactive selon la revendication 10, dans laquelle la lentille est formée à partir d'un polycarbonate uu d'un méthacrylate ou acrylate de polyalkyle.

12. Lentille photoréactive, ophthalmique ou plane, comprenant un mélange de composés héliochromiques tels que revendiqués dans la revendication 1 ou dans la revendication 2, en revêtement sur une de ses surfaces, ou laminé ou incorporé dans la matière de la lentille.

**Revendications pour l'Etat conctractant: AT**

1. Procédé de préparation d'un composé ayant la formule générale:

(I) 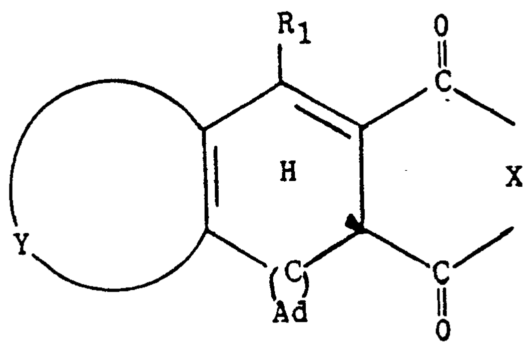

comprenant le fait de soumettre un composé de formule générale (IV) suivante à un chauffage dans un solvant jusqu'à une température dépassant 100°C ou à une radiation ultraviolette et à un chauffage concomitants, ou à un traitement avec un acide de Lewis, et de récupérer le produit de réarrangement:

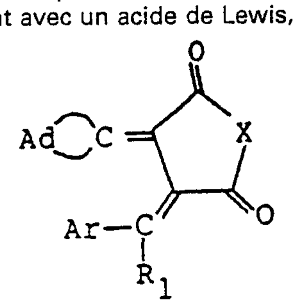 (IV)

dans lequel Ad⁀C< représente un groupe adamantylidène, $R_1$ représente hydrogène, alkyle, aryle ou aralkyle, et Ar représente un anneau benzène ou un anneau hétérocyclique à 5 membres ou un anneau hétérocyclique à anneau benzénique contenant oxygène ou soufre et X représente oxygène ou $>NR_2$ dans lequel $R_2$ est hydrogène, aryle, alkyle ou aralkyle.

2. Procédé selon la revendication 1 dans lequel Ar représente un anneau benzène contenant un substituant 2,5-; 2,4- ou 3,5- diméthoxy ou un groupe 2- ou 3- furano ou thiophéno.

3. Procédé selon la revendication 2 dans lequel le groupe furano ou thiophéno a un noyau benzénique ou est substitué avec un alkyle ou phényle inférieur.

4. Lentille photoréactive, ophthalmique ou plane, ayant un composé héliochromique en revêtement sur une de ses surfaces, ou laminé ou incorporé dans la matière de la lentille, le composé héliochromique ayant la propriété de former de manière réversible une forme structurelle colorée en étant irradié par la lumière ultraviolette et de revenir en une forme structurelle incolore ou plus pâle à la température ambiante normale en l'absence de stimulation ultraviolette, la forme colorée ayant la structure (a) suivant:

(a)

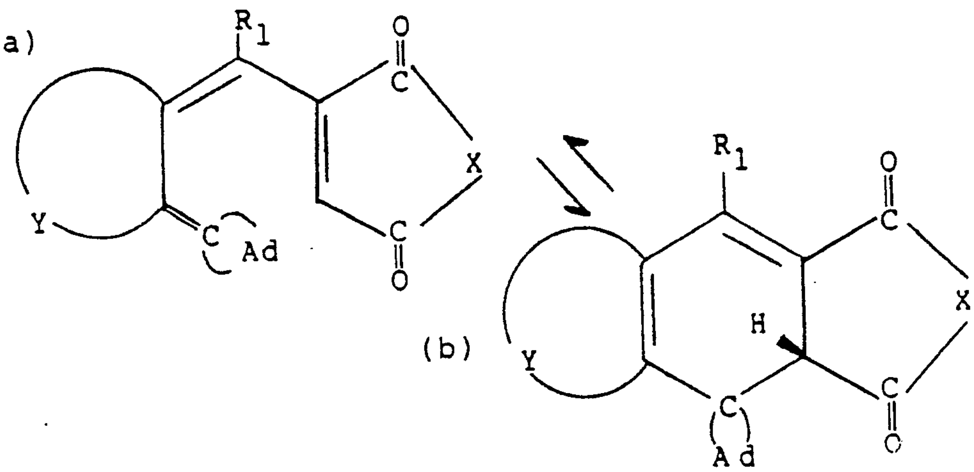

(b)

et étant capable de conversion en sa forme (b) incolore ou plus pâle par formation d'un anneau central à 6 membres, dans lequel

Ad⁀C< représente un groupe adamantylidène , $R_1$ représente hydrogène, alkyle, aryle ou aralkyle; X représente oxygène ou $>NR_2$ dans lequel $R_2$ est hydrogène, aryle, alkyle ou aralkyle et

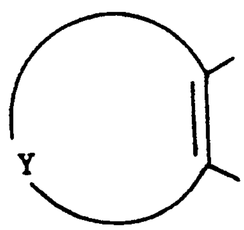

représente un groupe aromatique, un groupe hétérocyclique insaturé ou un groupe hétérocyclique à anneau benzénique.

5. Lentille photochromique, ophthalmique ou plane, ayant un composé héliochromique en revêtement sur une des ses surfaces ou laminé ou incorporé dans la matière de la lentille, le composé héliochromique ayant la formule générale suivante:

(I)

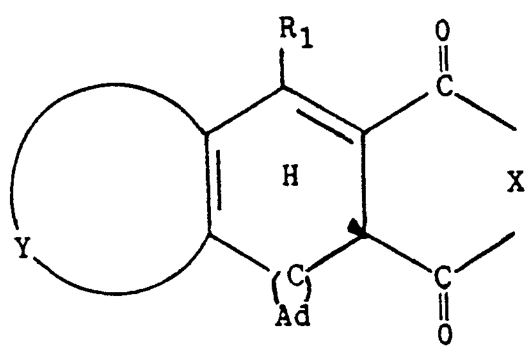

dans laquelle $R_1$, X et Ad⁀C<ont la même signification que dans la revendication 1, et

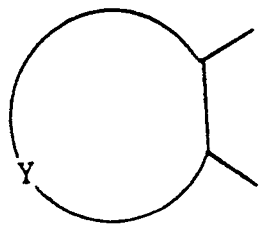

est dérivé d'un groupe 2,5-; 2,4- ou 3,5- diméthoxybenzo ou fait partie d'un groupe 2- ou 3-furano ou d'un groupe 2- ou 3-thiophéno.

6. Lentille photoréactive selon la revendication 5, dans laquelle la lentille est formée à partir d'un polycarbonate ou d'un méthacrylate ou acrylate de polyalkyle.

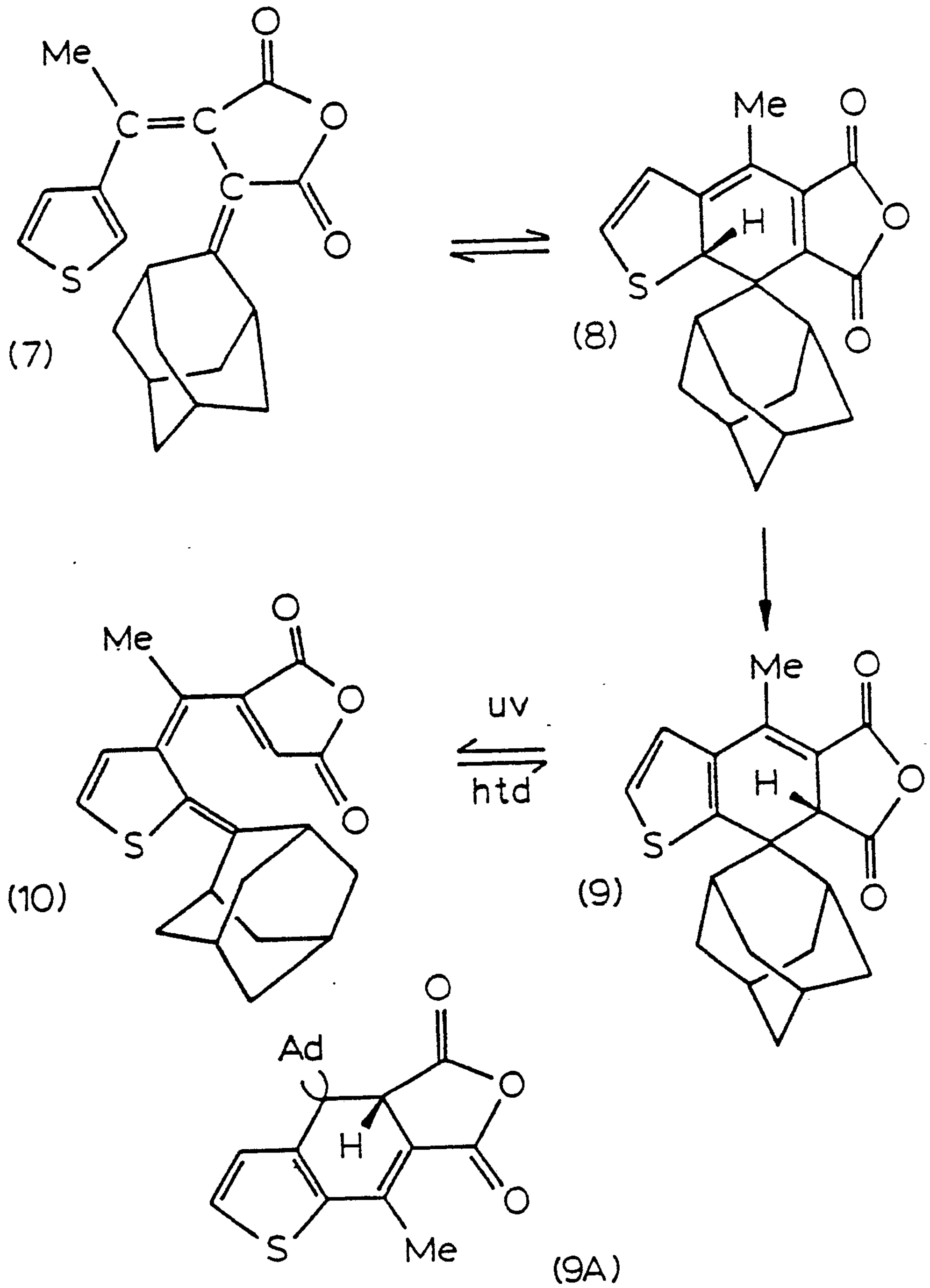


(7)

(8)

(10)

(9)

(9A)

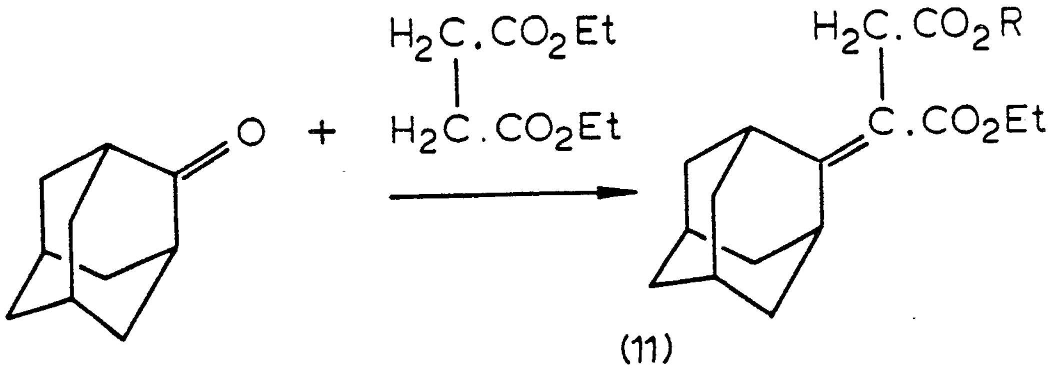

(11)

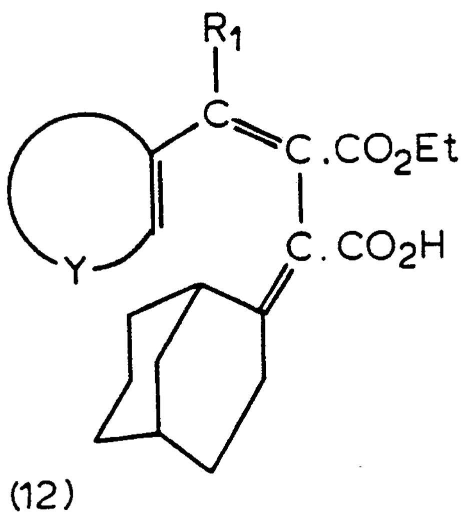

(12)

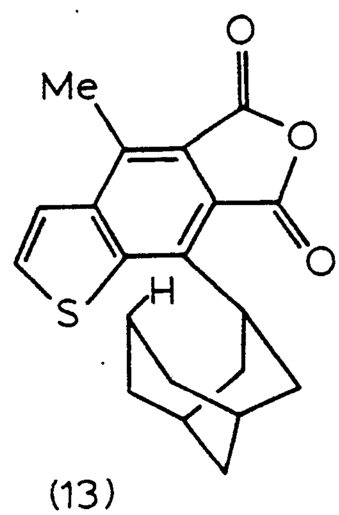

(13)

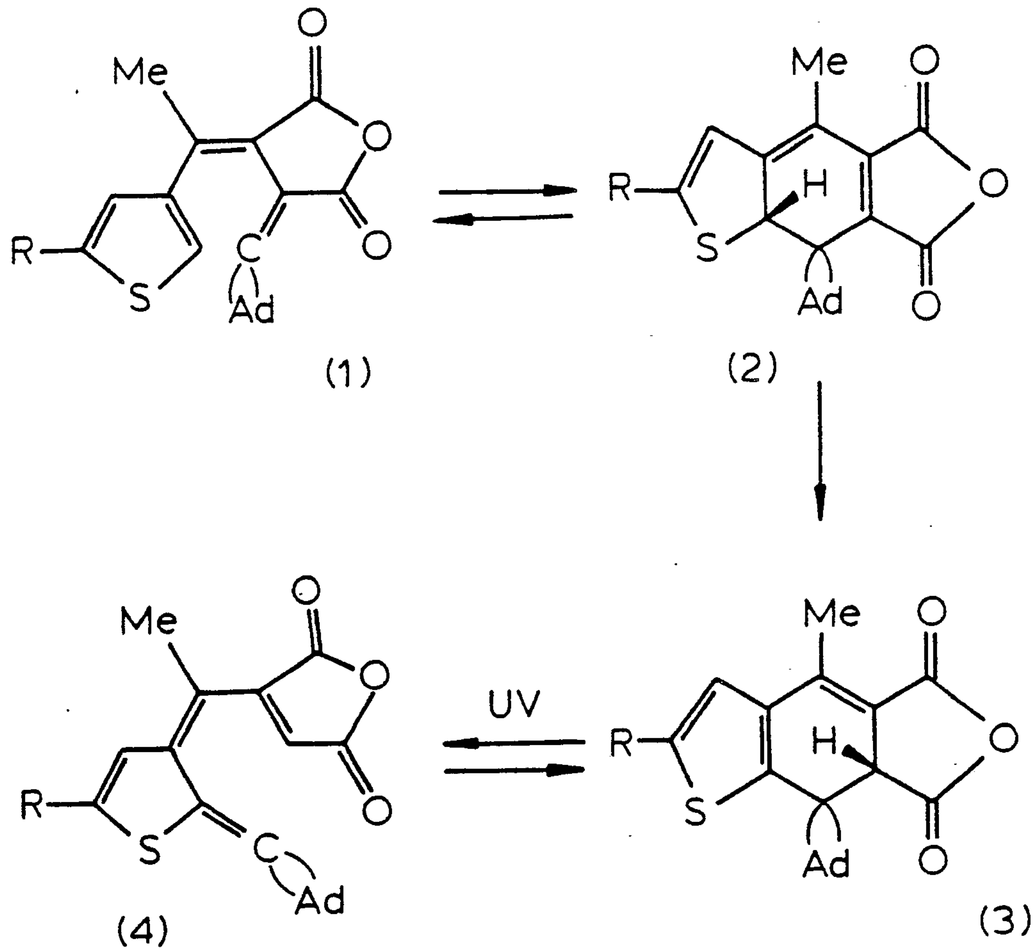


(1) (2) (3) (4)

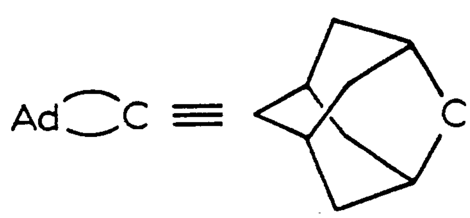

Examples 1 R = Ph
2 R = H
3 R = Br
4 R = $NO_2$

Scheme 1

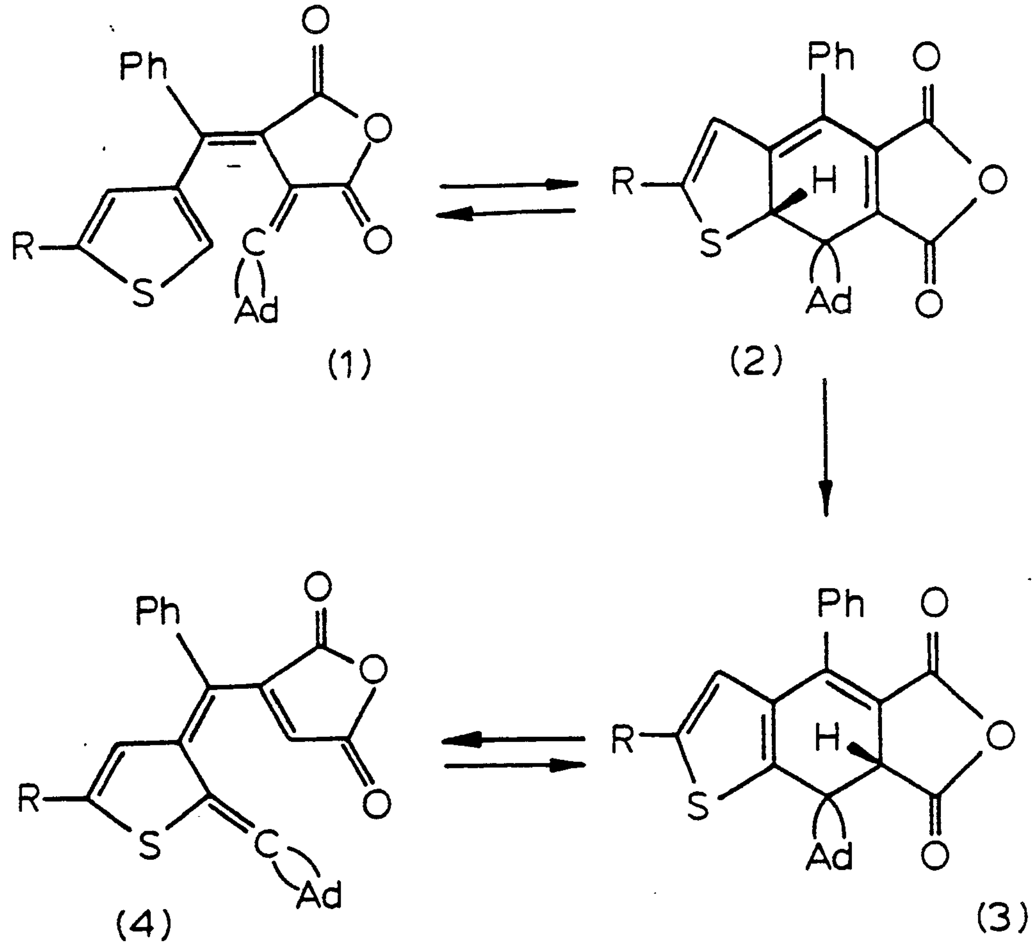

(1) (2)

(4) (3)

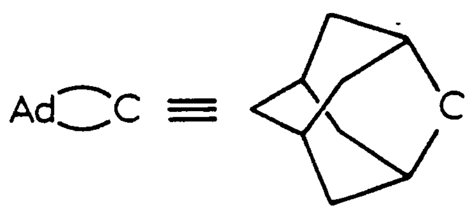

Example 5 R = H
6 R = Ph

Scheme 2

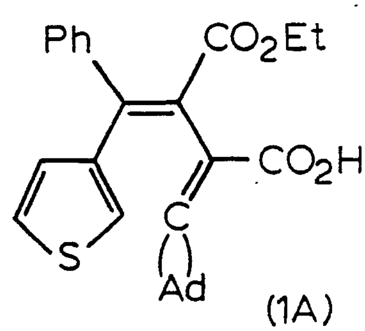

(1A)

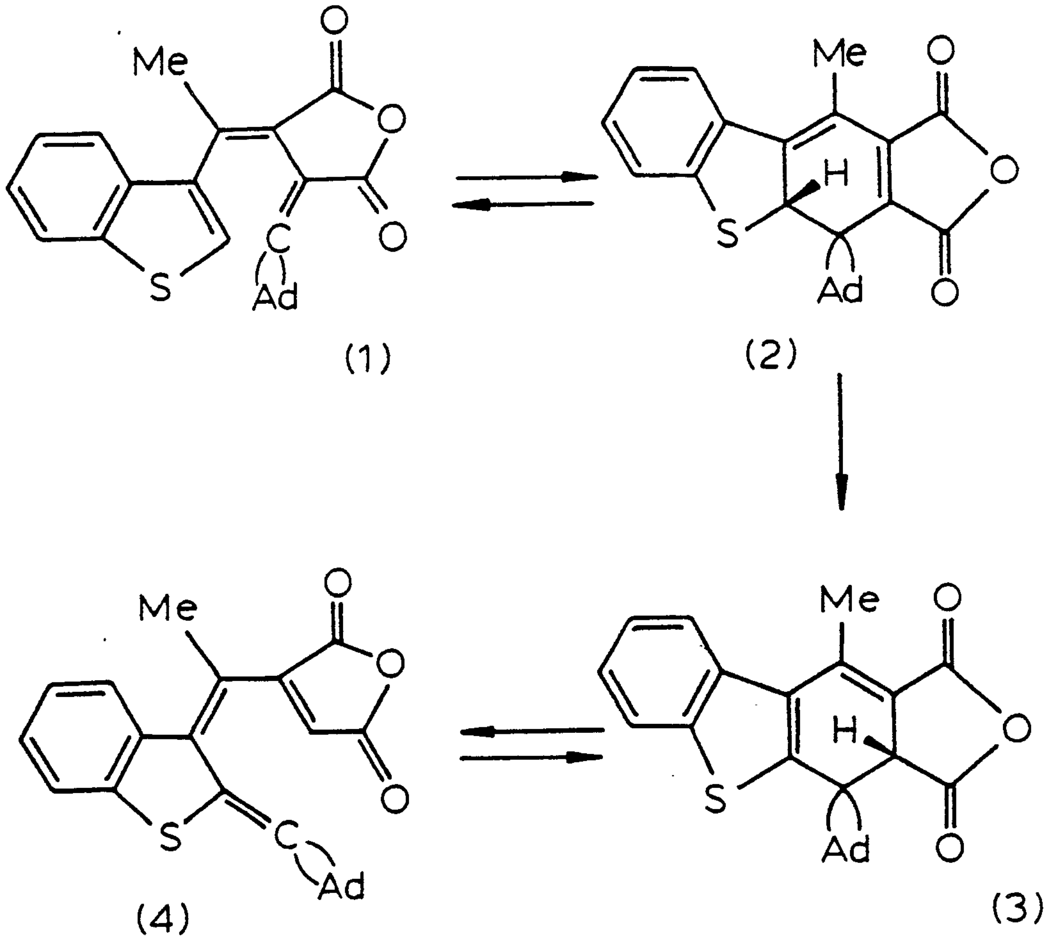

(1) (2)

(4) (3)

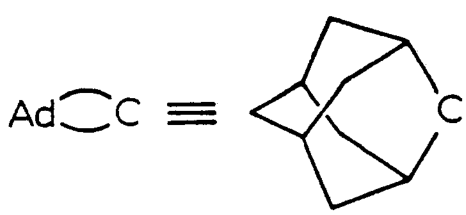

Scheme 3

Example 7

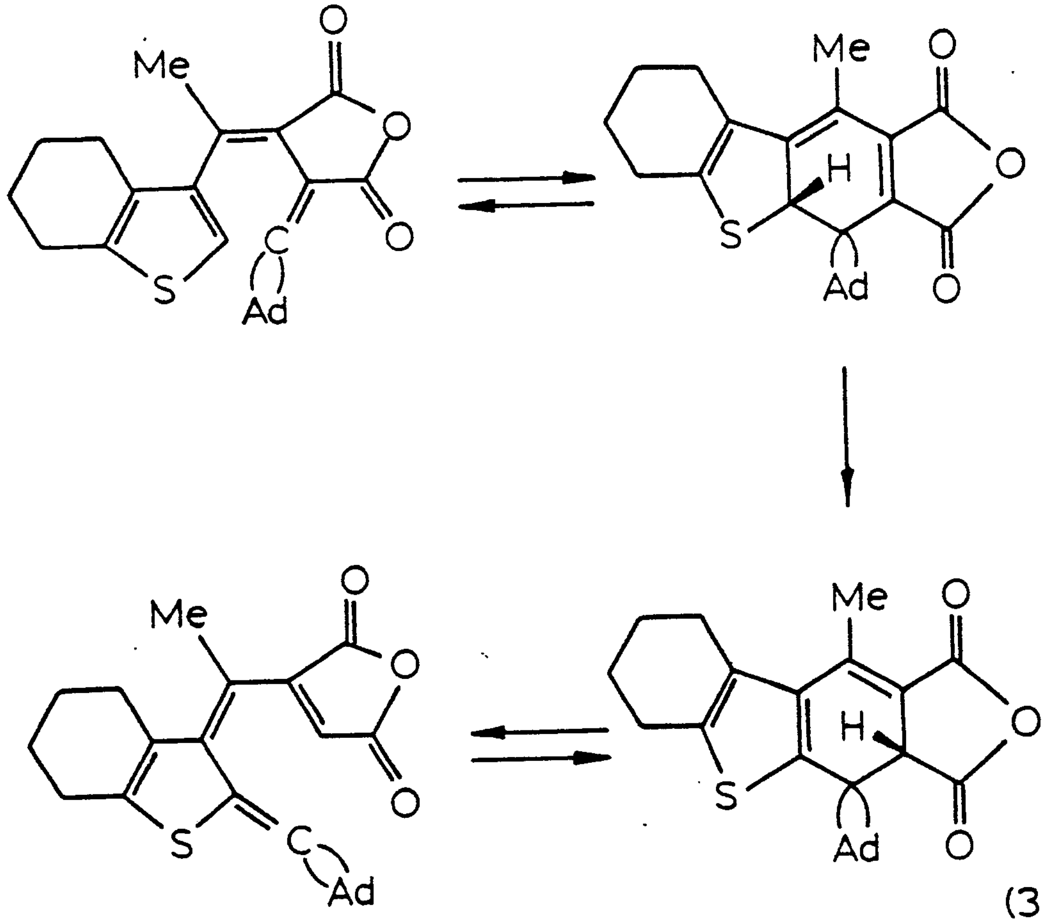

(3)

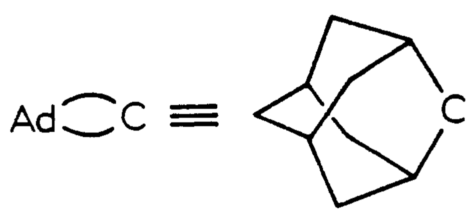

Scheme 4

Example 8

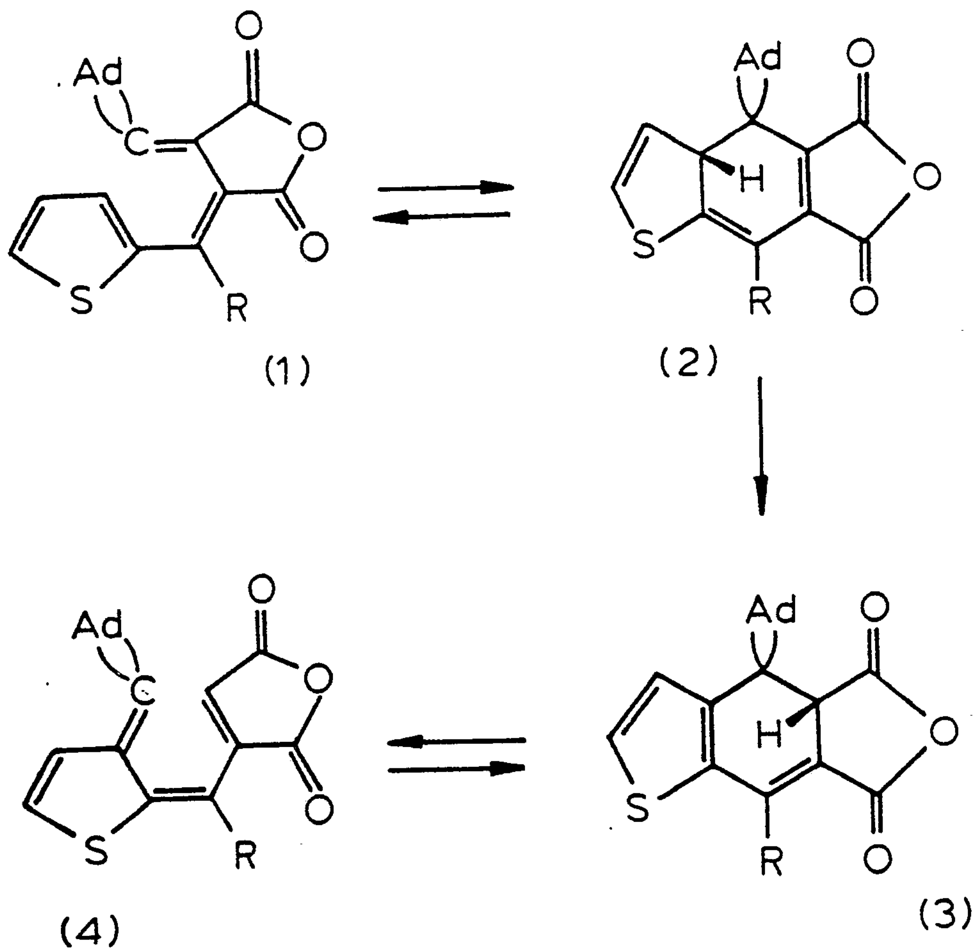

(1) (2) (3) (4)

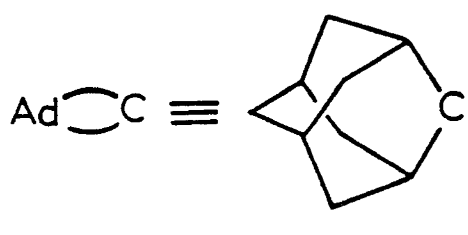

Scheme 5

Example 9 R = Me
10 R = Ph
11 R = p-MeO.$C_6H_4$

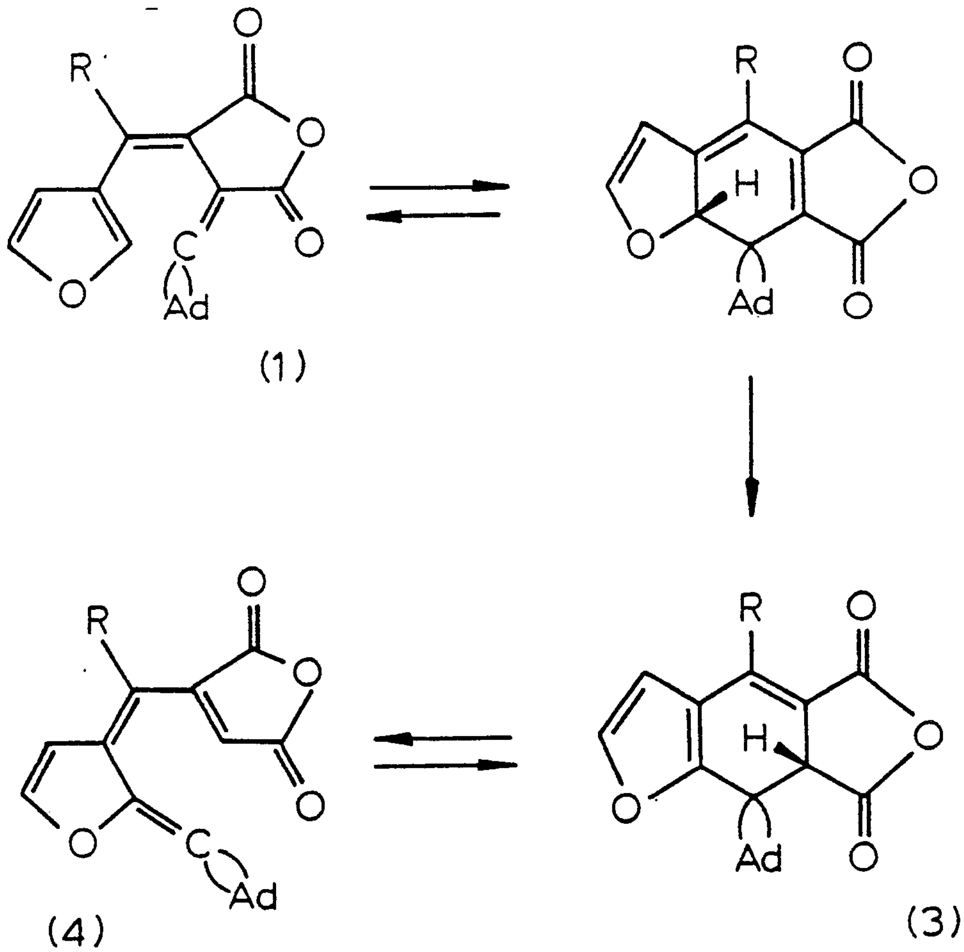

(1)

(4)

(3)

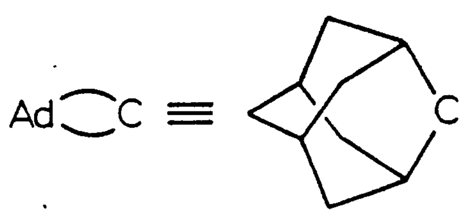

Scheme 6

Examples 12 R = Me

13 R = Ph

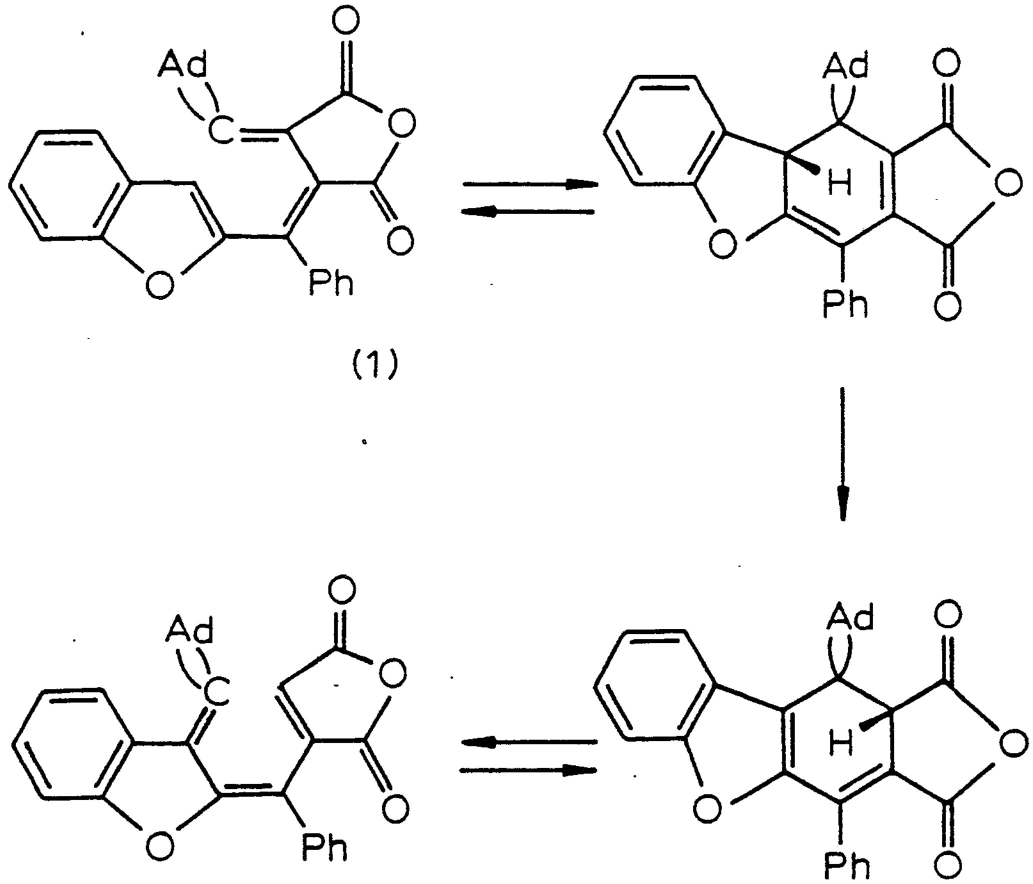

(1)

(4)

(3)

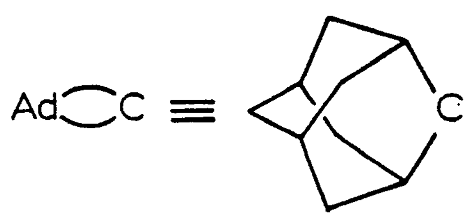

Scheme 7

Example 14

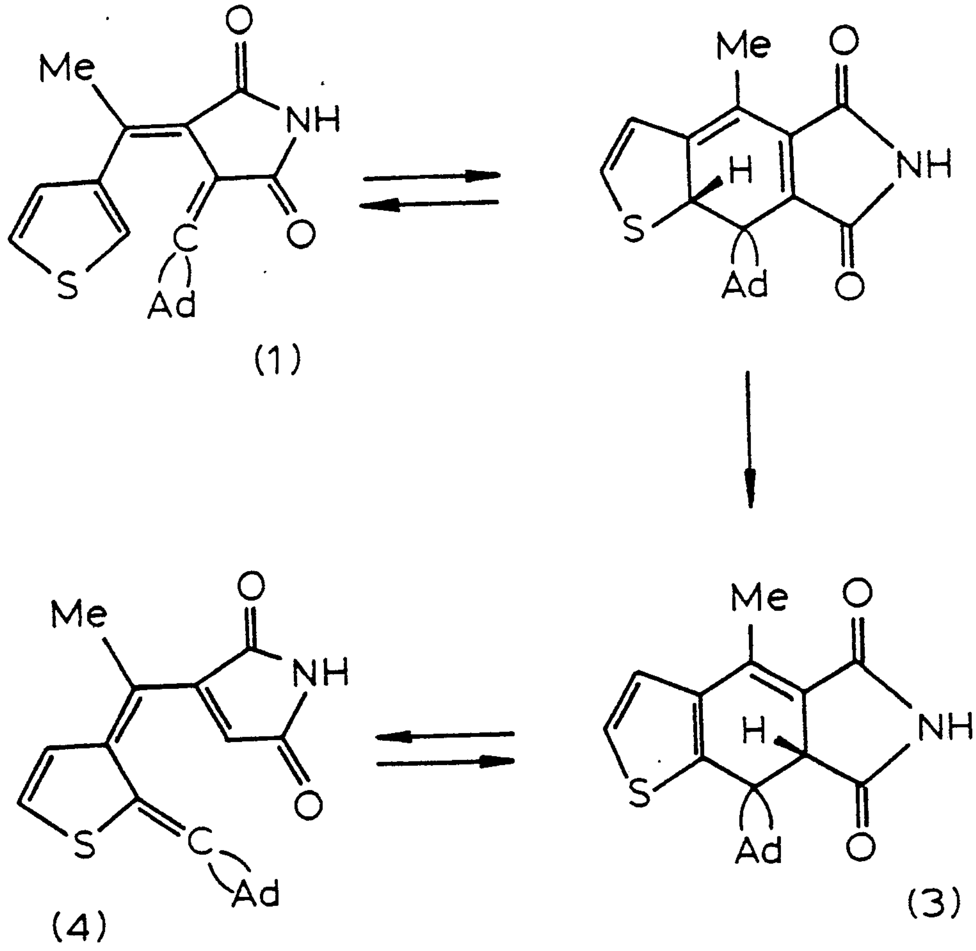

(1)

(4)

(3)

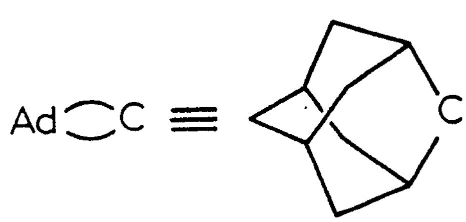

Scheme 8

Example 15

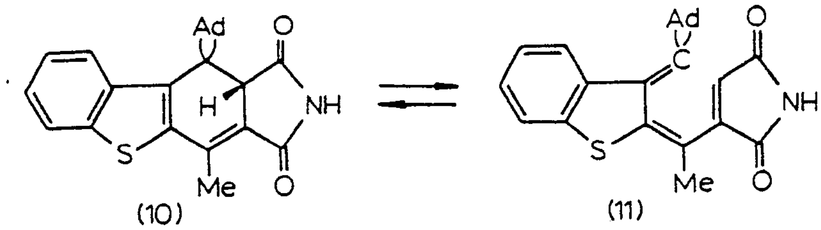

(10) (11)

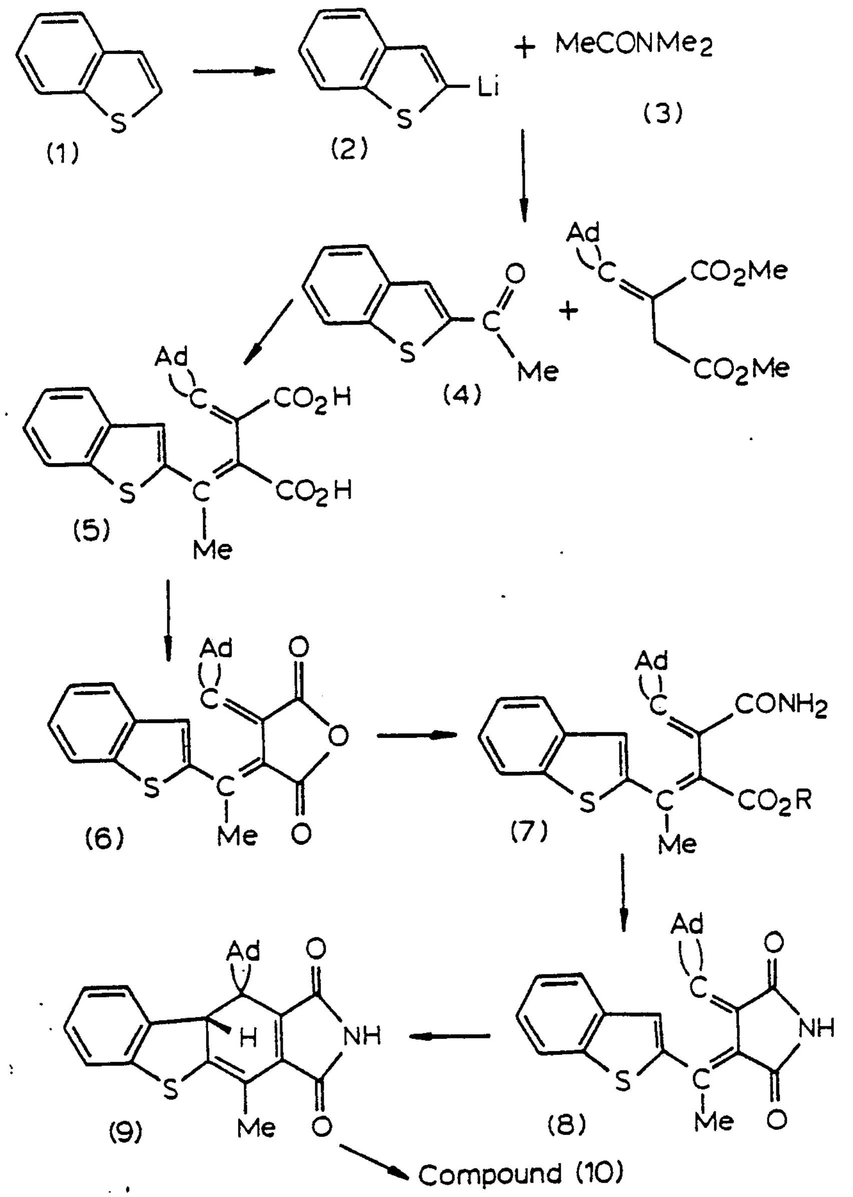

(1) (2) (3) (4) (5) (6) (7) (8) (9)



Example 16

Scheme 9

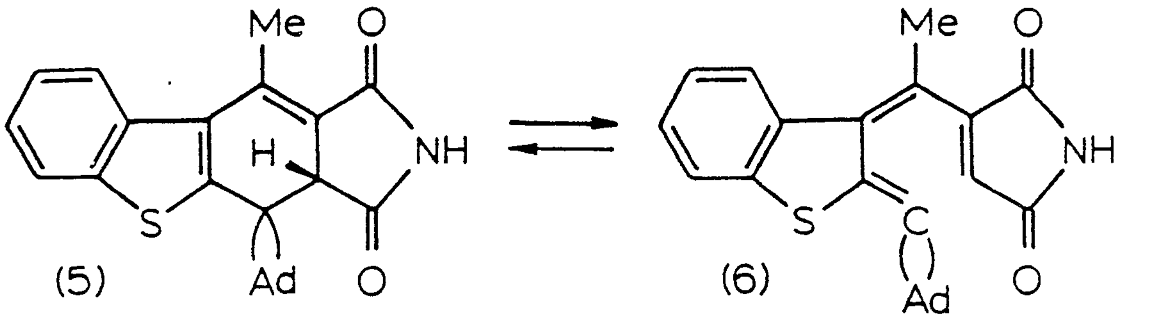

(5) (6)

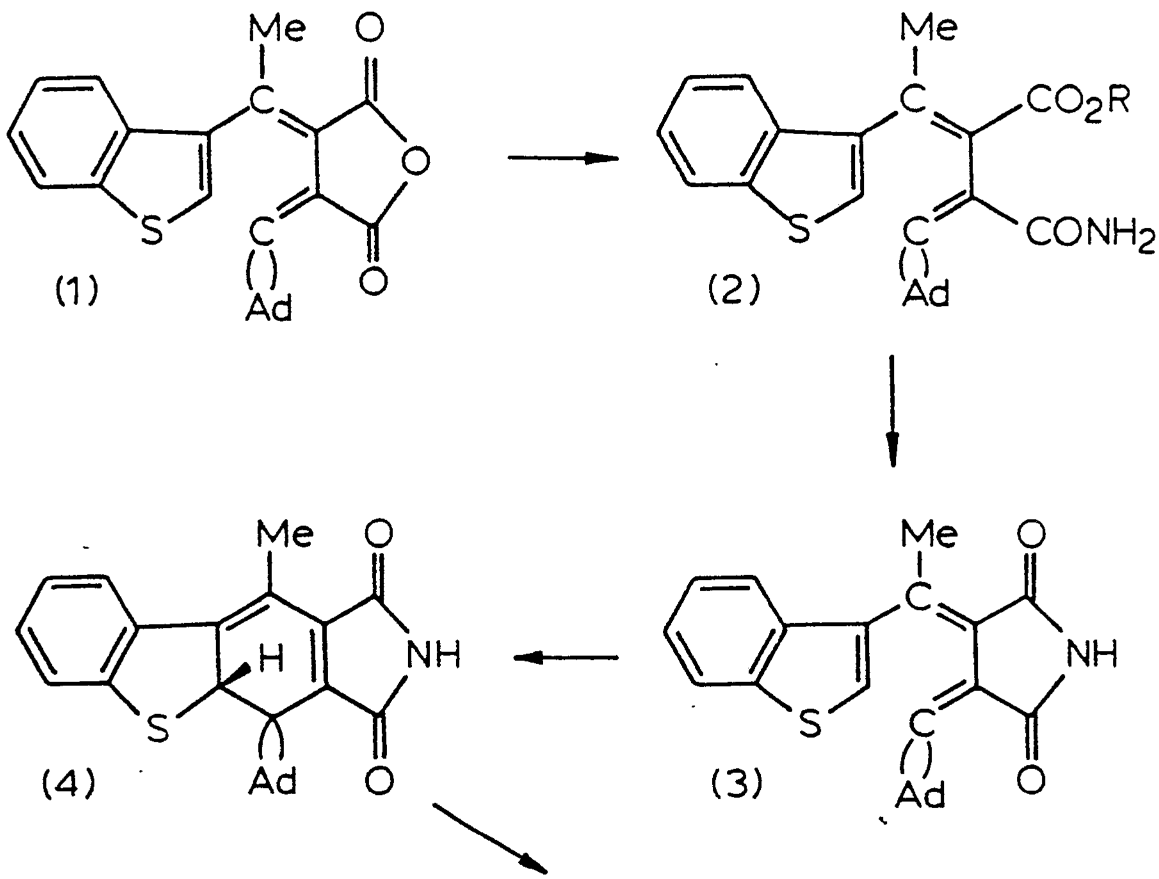

(1) (2) (4) (3)

Scheme 10

Example 17

<u>Reaction Scheme 11</u>

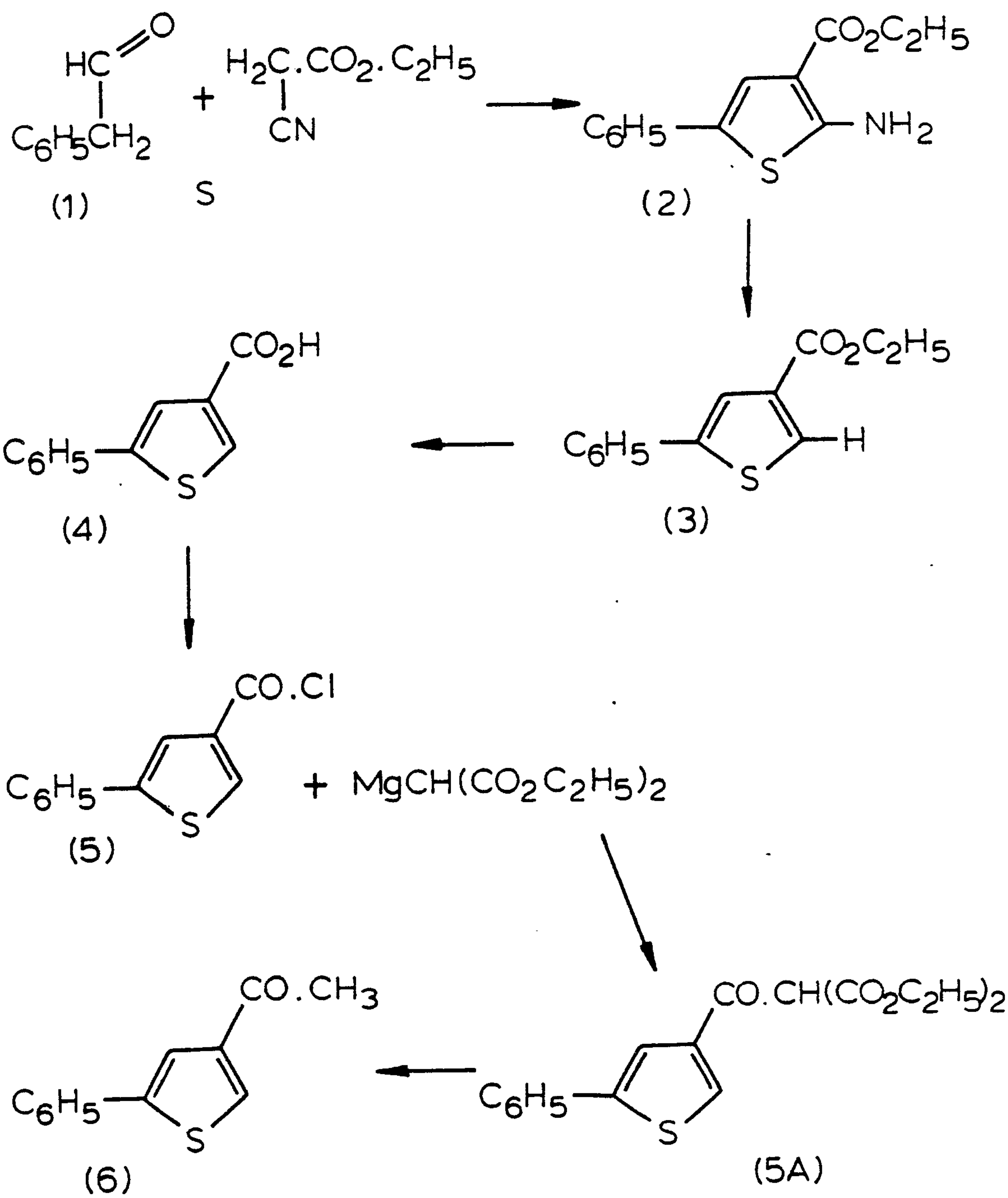

(1) (2) (3) (4) (5) (5A) (6)